(19) Europäisches Patentamt
European Patent Office
Office européen des brevets

(11) **EP 1 773 395 B1**

(12) **EUROPEAN PATENT SPECIFICATION**

(45) Date of publication and mention of the grant of the patent:
**22.06.2011  Bulletin 2011/25**

(21) Application number: **05775596.9**

(22) Date of filing: **29.07.2005**

(51) Int Cl.:
*A61K 45/00* (2006.01)  *A61K 38/21* (2006.01)

(86) International application number:
**PCT/US2005/026819**

(87) International publication number:
**WO 2006/015137 (09.02.2006 Gazette 2006/06)**

(54) **A METHOD FOR MANAGING CHOLESTEROL WITH A SERUM-FREE AND MITOGEN FREE CYTOKINE MIXTURE**

VERFAHREN ZUM VERWALTEN VON CHOLESTERIN MIT EINER SERUMS- UND MITOGENFREIEN ZYTOKIN-MISCHUNG

PROCEDE DE CONTROLE DU CHOLESTEROL AVEC UN MELANGE DE CYTOKINES EXEMPT DE SERUM ET EXEMPT DE MITOGENE

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.07.2004  US 592121 P**

(43) Date of publication of application:
**18.04.2007  Bulletin 2007/16**

(73) Proprietor: **Cel-Sci Corporation
Vienna, VA 22182 (US)**

(72) Inventors:
 • **KERSTEN, Geert
  McLean, VA 22101 (US)**
 • **TALOR, Eyal
  Baltimore, MD 21209 (US)**

(74) Representative: **Zwicker, Jörk et al
Dr. Volker Vossius
Patent- und Rechtsanwaltskanzlei
Geibelstrasse 6
81679 München (DE)**

(56) References cited:
**EP-A1- 0 374 791    WO-A2-02/083076
US-A- 5 591 772    US-A- 5 632 983
US-A- 5 945 097    US-B2- 6 896 879**

• **ROZENZWEIG I.B. ET AL.: 'Effects of Interleukin-2 (IL-2) on human plasma lipid, lipoprotein, and C-reactive Protein' BIOTHERAPY vol. 2, no. 3, 1990, pages 193 - 198, XP008073802**
• **DALEKOS G.N. ET AL.: 'Increased Serum Levels of Interleukin-1beta in the Systemic Circulation of Patients with Essential Hypertension: Additional Risk Factor for Antherogenesis in Hypertensive Patients' J LAB CLIN MED vol. 129, no. 3, March 1997, pages 300 - 308, XP003004467**

**Description**

INTRODUCTION

The present invention relates to a serum-free and mitogen-free mixture comprised of specific ratios of cytokines IL-1β, TNF-α, IFN-γ and GM-CSF to Interleukin 2 (IL-2) such as Leukocyte Interleukin Injection (LI) or Multikine ® and methods for treating or preventing a disease or disorder, for example, cardiovascular disease, dyslipidemia; dyslipoproteinemia; hyperlipidemia; a disorder of glucose metabolism; Syndrome X; a peroxisome proliferator activated receptor-associated disorder; obesity; hypertension; and renal disease.

BACKGROUND OF THE INVENTION

The evidence linking elevated serum cholesterol to coronary heart disease (CHD) is overwhelming with CHD being the leading cause of death in American men and women. Obesity, hyperlipidemia, dyslipidemia, dyslipoproteinemia; and diabetes have all been shown to play a causal role in atherosclerotic cardiovascular disease. Further, one human disease, termed "Syndrome X" or "Metabolic Syndrome", is manifested by defective glucose metabolism (insulin resistance), elevated blood pressure (hypertension), and a blood lipid imbalance (dyslipidemia). See Reaven, 1993, Annu. Rev. Med. 44:121-131.

Circulating cholesterol carried by plasma lipoproteins, which are particles of complex lipid and protein composition that transport lipids in the blood, which contributes to disease. Low density lipoprotein (LDL) and high density lipoprotein (HDL) are the major cholesterol-carrier proteins. LDL is believed to be responsible for the delivery of cholesterol from the liver, where it is synthesized or obtained from dietary sources, to extrahepatic tissues in the body. The term "reverse cholesterol transport" describes the transport of cholesterol from extrahepatic tissues to the liver, where it is catabolized and eliminated. It is believed that plasma HDL particles play a major role in the reverse transport process, acting as scavengers of tissue cholesterol. HDL is also responsible for the removal of non-cholesterol lipid, oxidized cholesterol and other oxidized products from the bloodstream.

Atherosclerosis, for example, is a slowly progressive disease characterized by the accumulation of cholesterol within the arterial wall. Compelling evidence supports the belief that lipids deposited in atherosclerotic lesions are derived primarily from plasma apolipoprotein B (apo B)-containing lipoproteins, which include chylomicrons, VLDL, IDL and LDL. The apo B-containing lipoprotein, and in particular LDL, has popularly become known as the "bad" cholesterol. In contrast, HDL serum levels correlate inversely with coronary heart disease. Indeed, high serum levels of HDL are regarded as a negative risk factor. It is hypothesized that high levels of plasma HDL are not only protective against coronary artery disease, but may actually induce regression of atherosclerotic plaque (e.g., see Badimon et al., 1992, Circulation 86: (Suppl. 111)86-94; Dansky and Fisher, 1999, Circulation 100: 1762-3.). Thus, HDL has popularly become known as the "good" cholesterol.

Cholesterol Transport

The fat-transport system can be divided into two pathways: an exogenous one for cholesterol and triglycerides absorbed from the intestine and an endogenous one for cholesterol and triglycerides entering the bloodstream from the liver and other non-hepatic tissue.

In the exogenous pathway, dietary fats are packaged into lipoprotein particles called chylomicrons, which enter the bloodstream and deliver their triglycerides to adipose tissue for storage and to muscle for oxidation to supply energy. The remnant of the chylomicron, which contains cholesteryl esters, is removed from the circulation by a specific receptor found only on liver cells. This cholesterol then becomes available again for cellular metabolism or for recycling to extrahepatic tissues as plasma lipoproteins.

In the endogenous pathway, the liver secretes a large, very-low-density lipoprotein particle (VLDL) into the bloodstream. The core of VLDL consists mostly of triglycerides synthesized in the liver with a smaller amount of cholesteryl esters either synthesized in the liver or recycled from chylomicrons. Two predominant proteins are displayed on the surface of VLDL, apolipoprotein B-100 (apo B-100) and apolipoprotein E (apo E), although other apolipoproteins are present, such as apolipoprotein CM (apo CIII and apolipoprotein CII (apo CII). When a VLDL reaches the capillaries of adipose tissue or of muscle, its triglyceride is extracted. This results in the formation of a new kind of particle called intermediate-density lipoprotein (IDL) or VLDL remnant decreased in size and enriched in cholesteryl esters relative to a VLDL, but retaining its two apoproteins.

In human beings, about half of the IDL particles are removed from the circulation quickly, generally within two to six hours of their formation. This is because LDL particles bind tightly to liver cells, which extract IDL cholesterol to make new VLDL and bile acids. The IDL not taken up by the liver is catabolized by the hepatic lipase, an enzyme bound to the proteoglycan on liver cells. Apo E dissociates from LDL as it is transformed to LDL. Apo B-100 is the sole protein of LDL.

Primarily, the liver takes up and degrades circulating cholesterol to bile acids, which are the end products of cholesterol metabolism. The uptake of cholesterol-containing particles is mediated by LDL receptors, which are present in high concentrations on hepatocytes. The LDL receptor binds both apo E and apo B-100 and is responsible for binding and removing both IDL and LDL from the circulation. In addition, remnant receptors are responsible for clearing chylomicrons and VLDL remnants (i.e., LDL). However, the affinity of apo E for the LDL receptor is greater than that of apo B-100. As a result, the LDL particles have a much longer circulating life span than LDL particles; LDL circulates for an average of two and a half days before binding to the LDL receptors in the liver and other tissues. High serum levels of LDL, the "bad" cholesterol, are positively associated with coronary heart disease. For example, in atherosclerosis, cholesterol derived from circulating LDL accumulates in the walls of arteries. This accumulation forms bulky plaques that inhibit the flow of blood until a clot eventually forms, obstructing an artery and causing a heart attack or stroke.

Ultimately, the amount of intracellular cholesterol liberated from the LDL controls cellular cholesterol metabolism. The accumulation of cellular cholesterol derived from VLDL and LDL controls three processes. First, it reduces the cell's ability to make its own cholesterol by turning off the synthesis of HMG-CoA reductase, a key enzyme in the cholesterol biosynthetic pathway. Second, the incoming LDL-derived cholesterol promotes storage of cholesterol by the action of ACAT, the cellular enzyme that converts cholesterol into cholesteryl esters that are deposited in storage droplets. Third, the accumulation of cholesterol within the cell drives a feedback mechanism that inhibits cellular synthesis of new LDL receptors. Cells, therefore, adjust their complement of LDL receptors so that enough cholesterol is brought in to meet their metabolic needs, without overloading. See Brown & Goldstein, In, The Pharmacological Basis Of Therapeutics, 8th Ed., Goodman & Gilman, Pergamon Press, New York, 1990, Ch. 36, pp. 874-896.

High levels of apo B-containing lipoproteins can be trapped in the subendothelial space of an artery and undergo oxidation. The oxidized lipoprotein is recognized by scavenger receptors on macrophages. Binding of oxidized lipoprotein to the scavenger receptors can enrich the macrophages with cholesterol and cholesteryl esters independently of the LDL receptor. Macrophages can also produce cholesteryl esters by the action of ACAT. LDL can also be complexed to a high molecular weight glycoprotein called apolipoprotein(a), also known as apo(a), through a disulfide bridge. The LDL-apo(a) complex is known as Lipoprotein(a) or Lp(a). Elevated levels of Lp(a) are detrimental having been associated with atherosclerosis, coronary heart disease, myocardial infarction, stroke, cerebral infarction, and restenosis following angioplasty.

The second report of the National Cholesterol Education Program (NCEP) Expert Panel on Detection, Evaluation, and Treatment of High Blood Cholesterol in Adults reviewed medications available for treating elevated lipid concentrations to reduce CHD risk. Pharmacologic agents are recommended for consideration when diet and exercise fail to decrease serum lipids to specific targets. The available cholesterol-lowering agents include bile acid sequestrants such as cholestyramine and colestipol, niacin and HMG-CoA reductase inhibitors. (National Cholesterol Education Program Expert Panel. Second Report of the Expert Panel on Detection, Evaluation, and Treatment of High Blood Cholesterol in Adults. Circulation. 1994;89:1329-445.)

HMG-CoA reductase inhibitors include lovastatin, simvastatin, pravastatin, fluvastatin, atorvastatin, rivastatin, itavastatin, rosuvastatin, and other statins. Sequestrants include cholestyramine, colestipol, and dialkylaminoalkyl derivatives of a cross-linked dextran. Inhibitors of cholesterol absorption include ezetimibe and beta-sitosterol while acyl CoA:cholesterol acyltransferase inhibitors includes avasimibe.

Niacin is also known to effectively lower the serum concentrations of total cholesterol and triglycerides while raising HDL cholesterol. Trials indicate that niacin may reduce mortality rate in persons with CHD. It's potential adverse effects, such as flushing, itching, gastrointestinal distress, and liver toxicity, limit its use in some patients and may reduce its cost-effectiveness. Fibric acids do not reduce LDL-C substantially, although they do lower elevated triglyceride concentrations.

However, among the known therapeutics, HMG-CoA reductase inhibitors are preferred. They are highly effective in decreasing LDL-C and the most recommended drugs in this class shown to reduce the risk of death from CHD. Treatment with HMG-CoA reductase inhibitors improves overall survival and lower CHD risk in persons with elevated cholesterol with or without major cardiovascular disease.

The mechanism of action of HMG-CoA reductase inhibitors is the rate-limiting step in hepatic cholesterol synthesis. HMG-CoA is converted to mevalonic acid, a reaction catalyzed by the enzyme 3-hydroxy-3-methylglutaryl (HMG) coenzyme A reductase. HMG-CoA reductases exert a profound effect on blood cholesterol levels through a reduction in hepatic synthesis of VLDL cholesterol, the precursor of LDL. This action increases the synthesis of LDL receptors on the cellular membrane of both hepatic and extrahepatic tissues. Because the primary function of LDL receptors is to remove LDL from the circulation, the degree of LDL receptor increase appears to correlate with the degree of cholesterol reduction.

The medications packaged under the names Mevacor, Pravachol, Zocor, Lipator or Lescol block the production of cholesterol within cells, causing the cells to increase specific receptors on their surface that will take up LDL cholesterol particles from the blood. This effect is especially prominent in the liver, which is the organ that largely controls cholesterol in the body. As the number of LDL receptors increases, the levels of total and LDL cholesterol in the blood will go down.

The effects on blood lipids is such that the total cholesterol and LDL cholesterol are reduced 15 to 40 percent while the triglyceride levels may decline 10 to 15 percent and the HDL cholesterol levels may increase 5 to 10 percent. Although the drug appears to be safe and well tolerated, certain side effects are associated with its use such as GI upset, headache, and dizziness and skin rashes.

Elevation of AST and ALT has also been associated with statin use. Risk factors include preexisting elevated liver enzymes, patient history of liver disease, and significant alcohol use. Each of these factors is a contraindication to statin use. Elevation in LFTs due to statins is usually asymptomatic but anorexia, weakness, and/or abdominal pain may be present. Elevated LFTs usually fall to normal after discontinuation of the drug, but serious hepatoxicity is possible. Because of these risks, the product's labeling recommends monitoring of LFTs. After a baseline measurement, the recommendations are to check LFTs after six and 12 weeks of therapy and periodically thereafter. If persistent elevations in AST and/or ALT of greater than three times the upper limit of normal occur, the drug should be discontinued.

HMG-CoA reductase inhibitors interfere with cholesterol synthesis and lower circulating cholesterol levels and, as such, might theoretically blunt adrenal or gonadal steroid hormone production. Results of clinical trials with statins in males and postmenopausal females were inconsistent with regard to possible effects of the drug on basal steroid hormone levels. In a study of 21 males, the mean testosterone response to human chorionic gonadotropin was significantly reduced ($p < 0.004$) after 16 weeks of treatment with 40 mg of pravastatin. However, the percentage of patients showing a $\geq 50\%$ rise in plasma testosterone after human chorionic gonadotropin stimulation did not change significantly after therapy in these patients. The effects of HMG-CoA reductase inhibitors on spermatogenesis and fertility have not been studied in adequate numbers of patients. Statins may also have a negative effect on the pituitary-gonadal axis in pre-menopausal females.

CNS vascular lesions, characterized by perivascular hemorrhage and edema and mononuclear cell infiltration of perivascular spaces, were seen in dogs treated with a statin at a dose of 25 mg/kg/day, a dose that produced a plasma drug level about 50 times higher than the mean drug level in humans taking 40 mg/day. Similar CNS vascular lesions have been observed with several other drugs in this class. A chemically similar drug in this class produced optic nerve degeneration (Wallerian degeneration of retinogeniculate fibers) in clinically normal dogs in a dose-dependent fashion starting at 60 mg/kg/day, a dose that produced mean plasma drug levels about 30 times higher than the mean drug level in humans taking the highest recommended dose (as measured by total enzyme inhibitory activity). This same drug also produced vestibulocochlear Wallerian-like degeneration and retinal ganglion cell chromatolysis in dogs treated for 14 weeks at 180 mg/kg/day, a dose which resulted in a mean plasma drug level similar to that seen with the 60 mg/kg/day dose.

In a 2-year study in rats fed with statins at doses of 10, 30, or 100 mg/kg body weight, there was an increased incidence of hepatocellular carcinomas in males at the highest dose ($p < 0.01$). Although rats were given up to 125 times the human dose (HD) on a mg/kg body weight basis, serum drug levels were only 6 to 10 times higher than those measured in humans given 40 mg statin as measured by AUC. The oral administration of 10, 30, or 100 mg/kg (producing plasma drug levels approximately 0.5 to 5.0 times the human drug levels at 40 mg) of statin to mice for 22 months resulted in a statistically significant increase in the incidence of malignant lymphomas in treated females when all treatment groups were pooled and compared to controls ($p < 0.05$).

A chemically similar drug in this class was administered to mice for 72 weeks at 25, 100, and 400 mg/kg body weight, which resulted in mean serum drug levels approximately 3, 15, and 33 times higher than the mean human serum drug concentration (as total inhibitory activity) after a 40 mg oral dose. Liver carcinomas were significantly increased in high-dose females and mid- and high-dose males, with a maximum incidence of 90 percent in males. The incidence of adenomas of the liver was significantly increased in mid- and high-dose females. Drug treatment also significantly increased the incidence of lung adenomas in mid- and high-dose males and females. Adenomas of the eye Harderian gland (a gland of the eye of rodents) were significantly higher in high-dose mice than in controls.

In another study with HMG-CoA reductase inhibitor, there was decreased fertility in male rats treated for 34 weeks at 25 mg/kg body weight, although this effect was not observed in a subsequent fertility study when this same dose was administered for 11 weeks (the entire cycle of spermatogenesis, including epididymal maturation). In rats treated with this same reductase inhibitor at 180 mg/kg/day, seminiferous tubule degeneration (necrosis and loss of spermatogenic epithelium) was observed. Other similar drugs in this class caused drug-related testicular atrophy, decreased spermatogenesis, spermatocytic degeneration, and giant cell formation in dogs. The clinical significance of these findings is unclear.

Safety in pregnant women has not been established using statins. For example, Pravastatin was not teratogenic in rats at doses up to 1000 mg/kg daily or in rabbits at doses of up to 50 mg/kg daily. These doses resulted in 20x (rabbit) or 240x (rat) the human exposure based on surface area (mg/meter$^2$). However, in studies with another HMG-CoA reductase inhibitor, skeletal malformations were observed in rats and mice. There has been one report of severe congenital bony deformity, tracheoesophageal fistula, and anal atresia (Vater association) in a baby born to a woman who took another HMG-CoA reductase inhibitor with dextroamphetamine sulfate during the first trimester of pregnancy.

Statins are not administered to women of child-bearing potential but can be when such patients are highly unlikely to conceive and have been informed of the potential hazards.

Additionally, all statins may cause myopathies, including rare cases of rhabdomyolysis with resulting acute renal failure secondary to myoglobinuria. The risk of myopathy appears to be greater with combinations of a statin and certain other drugs, including gemfibrozil (5% incidence), niacin (2% incidence), and cyclosporin (30% incidence). Symptoms are any unexplained muscle pain, tenderness, especially if accompanied by malaise or fever. The drug should be discontinued if myopathy is suspected or if elevations in creatinine phosphokinase (CKP) greater than 10 times normal occur.

Cytokines have been used to reduce blood cholesterol levels by administration of iL-4 and GM-CSF (EP 0 374 791), iL-10 (US 5 945 097) or iL-9(WO 02/083076).

Therefore, there is a need for methods for treating or preventing a dyslipidemia; dyslipoproteinemia or hyperlipidemia without elevating AST or ALT levels in liver compromised patients. There is also a need for another method of managing cholesterol without the risk of myopathies, including rhabdomyolysis with the resulting acute renal failure secondary to myoglobinuria. There is also a need to provide for an alternative method for managing cholesterol other than HMG-CoA reductase inhibitors for the treatment of disease or disorder, for example, cardiovascular disease where liver disease is a contraindication. There is still also a need for method for treating a disorder of glucose metabolism; Syndrome X; a peroxisome proliferator activated receptor-associated disorder; obesity; hypertension; and renal disease.

SUMMARY OF THE INVENTION

The present invention is based, in part, on methods of and methods for treating or preventing a disease or disorder, for example, cardiovascular disease, dyslipidemia; dyslipoproteinemia; hyperlipidemia; a disorder of glucose metabolism; Syndrome X; a peroxisome proliferator activated receptor-associated disorder; obesity; hypertension; and renal disease with a serum-free and mitogen-free Leukocyte Interleukin Injection (LI) mixture or Multikine® comprised of specific ratios of cytokines IL-1$\beta$ to IL-2, TNF-$\alpha$ to IL-2, IFN-$\gamma$ to IL-2 and GM-CSF to IL-2.

In embodiments of the invention, a method is disclosed for a serum-free and mitogen-free cytokine mixture being administered three times a week over a two week period in a range from about 20 IU to 12000 IU wherein IU represent International Units for Interleukin-2 given in World Health Organization 1st International Standard for Human IL-2, 86/504. In another embodiment, the serum-free and mitogen-free cytokine mixture is administered five times a week over a three week period in a range from about 20 IU to 12000 IU wherein IU represent International Units for Interleukin-2 given in World Health Organization 1st International Standard for Human IL-2, 86/504.

Specific applications include administering half a total daily dose of a Leukocyte Interleukin, Injection (LI) or Multikine ® and administering the other half of the total daily dose five (5) times per week for three (3) weeks. The total daily dose can be 200, 400, 800, 1200, 1600, 2400, 3200, 4800, 8000, 9600 and 12000 IU/mL as IL-2 wherein IU represents International Units for Interleukin-2 given in World Health Organization 1st International Standard for Human IL-2, 86/504.

Another embodiment of the invention includes Leukocyte Interleukin, Injection (LI) having specific ratios of cytokine to interleukin 2 (IL-2) as follows: IL-1$\beta$ to IL-2 at a ratio range of 0.4 - 1.5, and preferably at 0.7+/- 0.1 (IL-1$\beta$/IL-2), TNF-$\alpha$ to IL-2 at a ratio range of 3.2 - 11.3, and preferably at 9.5+/- 1.8 (TNF-$\alpha$/IL-2), IFN-$\gamma$ to IL-2 at a ratio range of 1.5 - 10.9, and preferably at 6.0+/- 1.1 (IFN-$\gamma$/IL-2), and GM-CSF to IL-2 at a ratio range of 2.2 - 4.8, and preferably at 4.0+/- 0.5 (GM-CSF/IL-2).

In other specific applications, the serum-free and mitogen-free cytokine preparation or pharmaceutical composition has further different cytokines and other small biologically active molecules wherein the ratio of each of the small biologically active molecules to IL-2 is as follows: IL-3 to IL-2 in a ratio range of 0.38 - 0.68, preferably at 0.53+/- 0.15, IL-6 to IL-2 in a ratio range of 37.2 - 53.8, preferably at 46+/- 5.9, IL-8 to IL-2 in a ratio range of 261 - 561.5, preferably at 411+/-10.6, IL-1$\alpha$ to IL-2 in a ratio range of 0.56 - 0.94, preferably at 0.75+/- 0.19, IL-10 to IL-2 in a ratio range of 2.82 - 3.22, preferably at 3.0+/- 0.18, IL-16 to IL-2 in a ratio range of 1.16 - 2.84, preferably at 1.84+/-0.68, G-CSF to IL-2 in a ratio range of 2.16 - 3.78, preferably at 2.97+/- 0.81, TNF-$\beta$ to IL-2 in a ratio range of 1.17 - 2.43, preferably at 1.8+/- 0.63, MIP-1$\alpha$ to IL-2 in a ratio range of 15.7 - 37.16, preferably at 22.7+/- 7.0, MIP-1$\beta$ to IL-2 in a ratio range of 17.1- 28.5, preferably at 22.8+/- 5.7, a RANTES to IL-2 in a ratio range of 2.3 - 2.7, preferably at 2.5+/- 0.13, a EGF to IL-2 in a ratio range of 0.267 - 0.283, preferably at 0.275+/- 0.008, $PGE_2$ to IL-2 in a ratio range of 3.63 - 5.42, preferably at 4.5+/- 0.87 and $TxB_2$ to IL-2 in a ratio range of 23.47-25.13, preferably at 24.3+/- 0.83.

Other objects and advantages of the present invention are set forth in the following description. The accompanying drawings and tables, which constitute a part of the disclosure, illustrate and, together with the description, explain the principle of the invention. One of ordinary skill in the art will appreciate that other aspects of this invention will become apparent upon reference to the attached figures and the following detailed description.

DETAILED DESCRIPTION OF THE INVENTION AND PREFERRED EMBODIMENTS

The present invention is concerned with methods for treating or preventing a disease or disorder, for example, cardiovascular disease, dyslipidemia; dyslipoproteinemia; hyperlipidemia; a disorder of glucose metabolism; Syndrome X; a peroxisome proliferator activated receptor-associated disorder; obesity; hypertension; and renal disease with a serum-free and mitogen-free cytokine mixture comprised of specific ratios of IL-1$\beta$ to IL-2, TNF-$\alpha$ to IL-2, EFN-$\gamma$ to IL-2 and GM-CSF to IL-2. One such novel cytokine mixture is Leukocyte-Interleukin Injection (LI), or Multikine® which has demonstrated immuno-modulatory capabilities. The clinical significance of the immuno-suppression in cancer patients unexpectedly impacts methods of preventing cardiovascular disease, a disorder of glucose metabolism; Syndrome X; a peroxisome proliferator activated receptor-associated disorder; obesity; hypertension and in particular dyslipidemia, dyslipoproteinemia or hyperlipidemia.

Administering Leukocyte Interleukin, Injection (LI) doses of 200, 400, 800, 1200, 1600, 2400, 3200, 4800, 8000, 9600 and 12000 IU/mL peritumorally three(3) times a week for two (weeks) or five (5) times per week for three (3) weeks altered the cholesterol level in patients initially treated with LI for head and neck cancer. The peritumoral dose during cancer treatment was performed by injecting, for example 100 IU/mL as IL-2 into four different sites around a tumor mass. Each of the four sites was injected with one quarter (1/4) of the entire peritumoral dose. The remaining half of the total dose was administered perilymphatically ipsilateral to the tumor site sequentially and on the same visit. The perilymphatic injections were given at one site at the posterior mandibular area in the area of the jugular lymphatic chain ipsilateral to the injected tumor mass.

Leukocyte-Interleukin Injection (LI), itself, is a serum-free, mitogen-free, antibiotic-free preparation produced from human peripheral blood mononuclear cells that include T-cells, B cells and macrophages. There are three "families" of cytokines in LI that together are important to the unique biological activity of LI. They include direct cytotoxic/cytostatic and virocidal/virostatic cytokines such as TNF-$\alpha$, and IFN-$\gamma$, lympho-proliferative cytokines such as IL-1, and IL-2 and chemotactic cytokines such as IL-6, IL-8 and MIP-1$\alpha$. Furthermore, the different cytokine and small biological molecules that constitute LI are all derived from the lectin (PHA) *in vitro* stimulation of human peripheral blood mononuclear cells that include T cells, B cells, and macrophages. Centrifugation on a Ficoll-Paque gradient separates the white blood cells (including T cells, B cells, and macrophages) from donor whole blood, and a series of washes (in physiologically buffered media) facilitates the isolation of lymphocytes, and the removal of red blood cells, cellular debris and other unwanted cellular components from the isolated white cell component of the whole donor blood.

LI contains different cytokines present at specific ratios of each cytokine to Interleukin 2 (IL-2) as follows: IL-1$\beta$ to IL-2 at a ratio range of 0.4 - 1.5, and preferably at 0.7+/- 0.1 (IL-1$\beta$/IL-2), TNF-$\alpha$ to IL-2 at a ratio range of 3.2 - 11.3, and preferably at 9.5+/- 1.8 (TNF-$\alpha$/IL-2), IFN-$\gamma$ to IL-2 at a ratio range of 1.5 -10.9, and preferably at 6.0+/- 1.1 (IFN-$\gamma$/IL-2), and GM-CSF to IL-2 at a ratio range of 2.2 - 4.8, and preferably at 4.0+/- 0.5 (GM-CSF/IL-2).

The remainder of the different cytokines and other small biologically active molecules in LI are also present within each preparation of the small biologically active molecule to IL-2 is as follows: IL-3 to IL-2 in a ratio range of 0.38 - 0.68, preferably at 0.53+/- 0.15, IL-6 to IL-2 in a ratio range of 37.2 - 53.8, preferably at 46+/- 5.9, IL-8 to IL-2 in a ratio range of 261 - 561.5, preferably at 411+/- 10.6, IL-1$\alpha$ to IL-2 in a ratio range of 0.56 - 0.94, preferably at 0.75+/- 0.19, IL-10 to IL-2 in a ratio range of 2.82 - 3.22, preferably at 3.0+/- 0.18, IL-16 to IL-2 in a ratio range of 1.16 - 2.84, preferably at 1.84+/-0.68, G-CSF to IL-2 in a ratio range of 2.16 - 3.78, preferably at 2.97+/- 0.81, TNF-$\beta$ to IL-2 in a ratio range of 1.17 - 2.43, preferably at 1.8+/- 0.63, MIP-1$\alpha$ to IL-2 in a ratio range of 15.7 - 37.16, preferably at 22.7+/- 7.0, MIP-1$\beta$ to IL-2 in a ratio range of 17.1- 28.5, preferably at 22.8+/- 5.7, a RANTES to IL-2 in a ratio range of 2.3 - 2.7, preferably at 2.5+/- 0.13, a EGF to IL-2 in a ratio range of 0.267- 0.283, preferably at 0.275+/- 0.008, PGE$_2$ to IL-2 in a ratio range of 3.63 - 5.42, preferably at 4.5+/- 0.87 and TxB$_2$ to IL-2 in a ratio range of 23.47 - 25.13, preferably at 24.3+/- 0.83.

Leukocyte-Interleukin Injection (LI) or Multiline® was tested using a characterization protocol and does not contain the following cytokines and other small biologically active molecules: IL-4, IL-7, and IL-15, TfR, sICAM, PDGF-AB, EFN-$\alpha$, EPO, LTC 4, TGF-$\beta$2, FGF basic, Angiogenin, sE-selectin, SCF, and LIF. LI contains only trace quantities (just above the level of detection of the assay) of IL-12, and LTB 4.

In the manufacturing process, mononuclear cells are separated from human donor "buffy coats" by step-gradient centrifugation and cultured with PHA to enhance production and secretion of IL-2 and other cytokines from the donor white blood cells in culture as disclosed in U.S. Patents 5,093,479, 4,390,623, 4,388,309, 4,406,830, 4,661,447, 4,681,844 and 4,464,355, all of which are incorporated herein by reference. Subsequently, the culture supernatant is aseptically harvested, clarified and subjected to a commercial virus exclusion process. The supernatant is then further concentrated approximately 10 fold by ultrafiltration and microfiltration.

At this point, Human Serum Albumin, Inj. USP is added and the concentrate is then buffered to a physiological pH and brought to a target IL-2 concentration per the label claim (example 400 IU/mL). The concentrate is then subjected to a second micro-filtration (0.22 micron-rated filter) and aseptically dispensed into sterile serum-type vials and labeled by its IL-2 content. Product potency is measured by the incorporation of radio-labeled thymidine by a cytotoxic T-lymphoid line (CTLL-2). The final injectable agent is further tested by ELISA for the presence of five marker cytokines: IL-2, IL-1β, GM-CSF, IFN-γ, and TNF-α.

Definitions

IL-2 - Interleukin 2 (IL-2): A 15.5-kD glycoprotein synthesized by CD4+ helper T lymphocytes (Formally known as T cell Growth Factor). IL-2 has an autocrine effect acting on the CD4+ T lymphocytes that produce it and on other cells of the immune system (including B lymphocytes, CD8+ T lymphocytes, NK [Natural Killer] cells and others).

IL-1β-Interleukin 1 beta (IL-1β): A 17-kD cytokine synthesized by activated mononuclear phagocytes is found in free form in the circulation and mediates inflammatory responses. It acts on CD4+ T lymphocytes to help facilitate their proliferation, and acts on B-lymphocytes as a growth and differentiation factor. It also induces the synthesis of IL-6 by mononuclear phagocytes.

TNF-α- Tumor Necrosis Factor alpha (TNF-α): A 157 amino acid (aa) residues protein, synthesized by stimulated monocytes, macrophages, B lymphocytes, T lymphocytes, an NK cells among others, found in a trimmeric form in the circulation. TNF mediates direct anti-tumor action, causing tumor cell lysis, facilitates leukocyte recruitment, inducing angiogenesis and promotes fibroblast proliferation.

IFN-γ- Interferon Gamma (IFN-γ): A 21-24-kD glycoprotein homodimer synthesized by activated T lymphocytes and NK cells, is a powerful activator of monocytes increasing monocytes ability to destroy intracellular microorganisms and tumor cells. It has direct anti-viral and anti-proliferative activity, and causes many cell types to express Class II MHC (Major Histocompatibility Complex) cell surface molecular complex, as well as increasing the expression of Class I MHC.

# GM-CSF – Granulocyte Macrophage - Colony Stimulating Factor (GM-CSF):

A 127 aa protein found as a monomer in the circulation, produced by macrophages and T lymphocytes, fibroblast and endothelial cells. It is a growth factor for hemopoietic cells, and stimulates the growth and differentiation of myelomonocytic lineage.

IL-3- Interleukin - 3 (IL-3): A 20-kD lymphokine synthesized by activated CD4+ T helper lymphocytes, acts as a colony-stimulating factor by facilitating the proliferation of some hematopoietic cells and promoting the proliferation and differentiation of T lymphocytes.

IL-6 - Interleukin - 6 (IL-6): A 26-kD cytokine produced by activated T lymphocytes, mononuclear phagocytes, endothelial cells, and fibroblasts. It acts on many cells but has a special function in enabling activated B-lymphocytes to differentiate into antibody secreting plasma cells, and induces hepatocytes to form acute-phase proteins (implicated in inflammatory responses) as well as fibrinogen.

IL-8 - Interleukin - 8 (IL-8): An 8-kD protein produced by macrophages and endothelial cells. Is a powerful chemotactic factor for neutrophils and T lymphocytes, and facilitates neutrophil adherence to endothelial cells.

IL-1α - Interleukin 1 alpha (IL-1a): A 17-kD cytokine (like IL-1β) is cleaved from a 33-kD precursor molecule, synthesized by activated mononuclear phagocytes, is rarely found in free form in the circulation and acts as a membrane-associated substance. It assists IL-1β in mediating inflammatory responses.

IL-10 - Interleukin - 10 (IL-10): An 18-kD polypeptide produced by CD4+ and CD 8+ T lymphocytes, monocytes, macrophages, activated B lymphocytes, and keratinocytes. It inhibits macrophages ability to present antigen particularly to $T_H$1-type cells, and secrete IL-6 and TNF.

IL-16- Interleukin - 16 (IL-16): A 14-kD tetrameric protein produced by CD8+ T lymphocytes, eosinophils, mast cells and respiratory epithelial cells. It has strong chemoattraction properties for CD4+ T lymphocytes and monocytes.

G-CSF - Granulocyte Colony Stimulating Factor (G-CSF): A 22 - 25-kD homodimer glycoprotein produced by macrophages, endothelial cells, fibroblasts and stromal cells. It increases granulocyte progenitor cells in the marrow, and sustains increase in blood neutrophils. It also enhances the ability of neutrophils to exhibit enhanced super-oxide production thought to be important in the destruction of microbially infected cells and tumor cells.

TNF-β- Tumor Necrosis Factor beta (TNF-β): A 25-kD protein produced by activated lymphocytes. It can kill tumor cells in culture, and stimulates proliferation of fibroblasts. In addition it mimics most of the other actions of TNF-α.

MIP-1α- Macrophage Inflammatory Protein - 1 alpha (MIP-1α): A 66-aa monomeric protein produced by macrophages and other cells. It is a chemo-attractant for monocytes, T lymphocytes and eosinophils.

RANTES - An 8-kD protein produced by T lymphocytes and is a chemo-attractant to monocytes, T lymphocytes and eosinophils, and promotes inflammation.

EGF - Epidermal Growth Factor (EGF): A trisulfated polypeptide of 53-aa residues. EGF is a member of the tyrosin kinase family, and has multiple functions including stimulation of the mitogenic response and assisting in wound healing.

PGE$_2$ - Prostaglandin E$_2$ (PGE$_2$): PGE$_2$ belong to a family of biologically active lipids derived from arachidonic acid through the cyclooxygenase enzymatic reaction. It is released by activated monocytes and blocks MHC Class II expression on T lymphocytes and macrophages.

TxB$_2$ - Thromboxane B$_2$ (TxB$_2$): TxB$_2$ is a member of biologically active compounds derived from polyunsaturated fatty acids by isomerization of prostaglandin and endoperoxidase PGH$_2$ via the enzyme thromboxane synthetase. TxB$_2$ has a physiological role in thromboembolic disease, and anaphylactic reactions.

IU (International Units) - A unit of measure of the potency of biological preparations by comparison to an international reference standard of a specific weight and strength *e.g.*, WHO 1st International Standard for Human IL-2, 86/504. International Units are the only recognized and standardized method to report biological activity units that are published and are derived from an international collaborative research effort.

U (Units as a measure of biological activity) - Shorthand for a variety of named "units", which each laboratory derives as a reference, which is further unique to the laboratory where the work is being performed. Each "unit" is different from one laboratory to another laboratory and is not a globally recognized standard such as International Units (IU).

USP - U.S. Pharmacopeia Monographs.

*P*-"p < 0.01": A term in mathematical statistics that denotes the level of probability of an event occurring under pre-set conditions.

ANOVA (Analysis of Variances) - A single factor analysis as described in Statistics and mathematical textbooks *e.g.,* "Handbook of Statistical Methods for Engineers and Scientists", Harrison M. Wadsworth, Jr., Ed., McGraw Hill 1990, and "Statistical Operations Analysis of Health Research Data", Robert P. Hirsch and Richard K. Riegelman, Eds. Blackwell Science Inc., 1996.

Mode of action and characterization of LI

Animal studies demonstrate that "mixed interleukins" have immunomodulatory and immunostimulatory activity *in vitro* (Hadden et al., "Mixed Interleukins and Thymosin Fraction V Synergistically Induce T Lymphocyte Development in Hydrocortisone-Treated Aged Mice", Cell. Immunol. 144:228-236 (1992)). Without being limited to any one theory, it is hypothesized that the local/regional injection of "mixed interleukins" overcomes local immuno-suppression. Subsequently, a break tolerance to tumor antigens occurs and allows for an effective local anti-tumor immune response to occur. It has also been known that the local instillation of interleukins in the region of the tumor or the actual transfection of Interleukin genes into a tumor markedly augments the anti-tumor immune response resulting in tumor regression as reported by Golumbek et al., "Treatment of Established Renal Cancer by Tumor Cells Engineered to Secrete Interleukin-4", Science 254:713-716 (1991).

However, data from several CEL-SCI trials pooled to investigate the potential effect of Leukocyte Interleukin Injections (LI) on other disease states demonstrated that serum cholesterol were reduced while keeping baseline AST/ALT values. A two-fold objective was applied for examining the data distribution to determine the correlation between Leukocyte Interleukin Injectoin (LI) or Multiline® treatment and a potential decrease in serum cholesterol and to determine whether the LI dose administered had a constant or differing effect on the cholesterol response pattern.

The distributions of the cholesterol values were first evaluated using univariate statistical methods, and plots of the data. Baseline concentrations of cholesterol were stratified protocol (and tested with an ANOVA model) to determine whether significant differences between protocols existed prior to pooling the data into a meta-database. Means with 95% confidence intervals were constructed for the aggregated data and various groups of the data. Means stratified by protocol, by dose, and protocol by dose, were compared for trends over time and for differences between dose groups. Regression analyses were conducted to determine whether the slope of the change measuring the average cholesterol concentrations, as a function of LI treatment, was statistically significant from zero. Generalized Linear models (Manova) were conducted to test whether dose level differences were statistically significant, and to test whether a patients initial cholesterol level, i.e. concentration prior to treatment, had a significant relationship with the response to Leukocyte Interleukin injection.

Patients

Information regarding the control Protocol Number, clinical study phase, clinical patient population studied, total number of weeks, per week administration and total serum cholesterol (and ALT/AST) measurement techniques are provided in Table 1.

Table 1

| | Protocol Number | Clinical Study Phase, and Clinical Patient population studied | Total Number of Weeks | per Week Administration and route of administration | Total Serum Cholesterol (and ALT/AST) Measurement |
|---|---|---|---|---|---|
| Cel- 01 | 33575-01 | Phase I/II Clinical Study in Recurrent Metastatic H&N Cancer Both Male/Female (M/F), all stages of disease which failed higher priority therapy (Multikine and CIZ*) | For 8 - 26 (weeks, if disease did not progress) | 3-5 per week (Peri-tumorally) | Standard Chemistry/Clinical Laboratory technique |
| Cel-02 | 50234-02 | Phase I/II Clinical Study in Advanced Primary H&N Cancer (Squamous Cell Carcinoma) Both M/F, (Stages as T2-3N0-2 M0) given with CIZ** | For 2 (prior to surgical resection of the tumor) | 3 times per week (½ of Multikine dose Peri-tumorally, ½ perilymphatically) | Standard Chemistry/Clinical Laboratory technique |
| Cel-04 | 50234-01 | Phase I/II Clinical Study in Advanced Primary H&N Cancer (Squamous Cell. Carcinoma) Both M/F, (Stages as T2-3N0-2 M0) given with CIZ** | For 2 (prior to surgical resection of the tumor) | 3 per week (Peri-tumorally) | Standard Chemistry/Clinical Laboratory technique |
| Hun-01 | 50234-03 | Phase I/II Clinical Study in Advanced Primary H&N Cancer (Squamous Cell Carcinoma) Both M/F, (Stages as T2-3N0-2 M0) given with CIZ** | For 2 (prior to surgical resection of the tumor) | 3 times per week (Peri-tumorally) | Standard Chemistry/Clinical Laboratory technique |

(continued)

| | Protocol Number | Clinical Study Phase, and Clinical Patient population studied | Total Number of Weeks | per Week Administration and route of administration | Total Serum Cholesterol (and ALT/AST) Measurement |
|---|---|---|---|---|---|
| Hun-02 | 50234-03 | Phrase II Clinical Study in Advanced Primary H&N Cancer (Squamous Cell Carcinoma) Both M/F, (Stages as T2-3N0-2 M0) given with CIZ** | For 3 (prior to surgical resection of the tumor) | 5 time per week (½ of Multikine dose Peri-tumorally, ½peri-lymphatically) | Standard Chemistry/Clinical Laboratory technique |

*CIZ= i.v. Cylophosphamide (low dose, 300 mg/M$^2$), Indomethacin (25mg, tid), and Zinc sulfate, 142 mg (po, qd, 50mg as elemental Zinc). CIZ was administered as follows, Cylophosphamide (iv), one time only, at three (3) days prior to the initial Multikine administration, Indomethacin (three times per day - with food, daily - staring with the first administration of Multikine to the last day of Multikine administration and Zinc Sulfate (the same as Indomethacin administration schedule) **CIZ= i.v. Cylophosphamide (low dose, 300 mg/M$^2$), Indomethacin (25mg, tid), and Zinc sulfate, 142 mg (po, qd, 50mg as elemental Zinc). CIZ was administered as follows, Cylophosphamide (iv), one time only, at three (3) days prior to the initial Multikine administration, Indomethacin (three times per day - with food, daily - staring with the first administration of Multiline to 24 hours before surgical resection of the tumor, and Zinc Sulfate (the same as Indomethacin administration schedule)

Treatment protocol

One protocol of Leukocyte Interleukin, Injection (LI) administration is performed in the following manner; the total daily dose is injected three to five times per week peri-tumorally. LI is administered intradermally at the circumferential margins of the visible/palpable tumor mass for a period ranging from eight to twenty six weeks.

A single intravenous infusion of cyclophosphamide is given, Inj., 300 mg/m$^2$. three days prior to the first LI administration. Indomethacin (25 mg) is self-administered orally (with food), three times daily for a total daily dose of 75 mg beginning 3 days post cyclophosphamide administration and for a first cycle wherein each cycle of treatment is two months. Zinc Sulfate (50 mg, as elemental Zinc) and multivitamin supplement, once daily, is self-administered beginning 3 days after cyclophosphamide administration.

Leukocyte Interleukin, Injection (LI) administration can also be performed in the following manner; the total daily is injected three to five times per week peritumorally. Alternatively, half the total LI dose can be administered peri-tumorally where 1/4 of the peritumoral dose [approximately 100 IU for 800 IU/mL total dose] is administered at each of four sites around the tumor mass. The remaining one half (400 IU/mL of IL-2 for 800 IU/mL total dose) is administered perilymphatically ipsilateral to the tumor site (sequentially and on the same visit) over a three week period, 5 times per week for three weeks. LI is administered intradermally at the circumferential margins of the visible/palpable tumor mass. The perilymphatic injections are given at the posterior mandibular area in the area of the jugular lymphatic chain ipsilateral to the injected tumor mass in protocols Cel-02 and Hun-02. In the other protocols, Cel-01 and Cel-04, the doses of LI were only administered peri-tumorally.

A single intravenous infusion of cyclophosphamide is given, Inj., 300 mg/m$^2$, three days prior to the first LI administration. Indomethacin (25 mg) is self-administered orally (with food), three times daily for a total daily dose of 75 mg beginning 3 days post cyclophosphamide administration and until 24 hours prior to surgery. Zinc Sulfate (50 mg, as elemental Zinc) and multivitamin supplement, once daily, is self-administered beginning 3 days after cyclophosphamide administration and until 24 hours prior to surgery.

Patient examination

Prior to the inclusion in the trial patients underwent a general assessment. Their medical history is also reviewed. Once enrolled a complete physical examination, hematological, blood chemistry workup, chest cardioradiography and electrocardiogram were performed. When possible imaging of the primary tumor site is done to obtain a baseline image. All patients have a baseline bi-dimensional measurement of their primary tumor by measuring the two major perpendicular diameters. Patients are interviewed at each subsequent visit and are asked about quality of life (e.g., level of pain, degree of tongue mobility, etc.) using a qualified questionnaire. The treating physician assessed toxicity at each visit.

Leukocyte Interleukin Injection (LI)

Leukocyte Interleukin Injection (LI) is prepared from selected human peripheral blood mononuclear cells obtained from the US Red Cross after passing all FDA mandated testing for blood for transfusion cultured with mitogen. No first time donors are allowed. The serum free culture supernatant is then aseptically harvested, clarified, subjected to a commercial virus exclusion process, concentrated, and microfiltered. Human Serum Albumin, Inj. USP is added to the concentrate and the resultant solution is buffered to physiological pH, brought to a target IL-2 concentration, and subjected to a second microfiltration. The formulated drug solution is aseptically dispensed into sterile serum-type vials and labeled by its content of IL-2. Product potency is measured by the incorporation of radiolabeled thymidine (in vitro) by the use of a cytotoxic T-lymphoid (CTLL-2), IL-2 dependent cell line. The final injectable agent is further tested by ELISA or the presence of five marker cytokines: IL-2, IL-1$\alpha$, GM-CSF, IFN-$\gamma$, and TNF-$\alpha$. LI is further subjected to quality control tests for sterility, bacterial endotoxins, pH, and total protein concentration, and other physical-chemical tests.

LI is provided frozen in a borosilicate glass serum vial containing 2.2 mL of drug at the label claim as IL-2 (400 IU/ml) for peritumoral, intratumoral, perilymphatic or subcutaneous administration. LI is subjected to quality control tests for identity, sterility, bacterial endotoxins, pH, and total protein concentration. Each vial is inspected for particulate contamination and appearance. The preparation has a total protein content of 3 mg/mL wherein the material is supplied sterile and pyrogen free. LI has an assigned expiration date of 24 months from date of manufacture when the drug is stored at -20˚C.

Cyclophosphamide

Cyclophosphamide, Inj. USP (Bristol-Myers-Squibb, UK) is supplied as a sterile powder containing 45 mg sodium chloride, 75 mg mannitol or approximately 82 mg sodium bicarbonate per 100 mg cyclophosphamide for reconstitution prior to intravenous infusion.

Indomethacin

Indomethacin USP (Sanofi - Synthelabo, France) is supplied as 25 mg tablets for oral self-administration with food.

Zinc Sulfate and Multivitamins

Zinc sulfate (50 mg as elemental Zinc, R. P. Scherer Corporation, Clearwater, Florida, USA) and OTC multi-vitamins are supplied by the clinic to each patient for self-administration.

Protocol differences

Data for cholesterol and Leukocyte Interleukin Injection (LI) from four different protocols Cel-01 (33575-01), Cel-02. (50234-02), Cel-04 (50234-01) and Hun-01 and Hun-2 both (50234-03) were combined for meta-analysis. These trials were designed as Phase I/II safety (and pilot efficacy) trials with ascending dose levels of Leukocyte Interleukin Injection on Neck, and Head and Neck Squamous Carcinoma.

The general design of the trials differed most significantly from a protocol Cel-01. In Cel-01, patients received an initial course of 2 weeks of Leukocyte Interleukin Injections (LI) alone followed by a brief washout period. The patient received additional courses of treatment, with a maximum of six courses, and remained in the study until disease progression or the completion of the six courses. Each course of LI treatment in this trial consisted of 21 days. The first course of treatment following the initial two week LI treatment also added CIZ to the LI treatment. See Table 1. The Cel-02, Cel-04, Hun-01 and Hun-02 studies were similar in protocol. Patients in these trials received injections 3 or 5 times a week in addition to Indomethacin, vitamins and zinc supplements. The active trial period did not exceed three weeks.

Dosing regimens, Leukocyte Interleukin injection frequency and the site of injection (tumoral, lymphatic) varied between and within protocol. Not all studies included multi-vitamins, zinc supplements or a dietary control (in-patient diet.) The trials were conducted in different countries, and had patients with different Head and Neck cancer stage and medical histories.

All studies included both men and women. In the analyses, a control for the gender mix of each trial population was not provided even though a gender difference in cholesterol levels and responses to medication generally exists. Cholesterol determinations were done at different laboratories with slightly different relative ranges. However, the laboratories and relative ranges stayed constant within protocol and trial period. Consequently, any bias due to laboratory differences was kept constant and therefore did not affect the precision of the estimate of change from baseline to endpoint.

Baseline Population Differences

Based on ANOVA test statistics, average baseline values for Cell-01 (33575-01) patients were significantly lower than the average baseline concentration in the higher dose groups of Cel-04 (50234-01) and Hun-02 (50234-03). Generally, however, the average baseline cholesterol concentrations averaged within a protocol population and compared across other protocols were statistically equivalent as show in Table 2.

Table 2
Baseline Cholesterol Average concentration mg/dl By Protocol & Treatment Dose

| Protocol | Dose | N | Mean | Std Dev |
|---|---|---|---|---|
| Cel-01 | 200 | 5 | 188.3 | 34.6 |
| Cel-01 | 400 | 3 | 196.6 | 20.1 |
| Cel-01 | 800 | 4 | 169.9 | 39.9 |
| Cel-01 | 1600 | 4 | 149.6 | 24.9 |
| Cel-02 | 800 | 9 | 202.5 | 36.7 |
| Cel-02 | 1600 | 5 | 204.1 | 16.2 |
| Cel-02 | 3200 | 12 | 214.1 | 33.8 |
| Cel-04 | 1200 | 6 | 227.8 | 43.6 |
| Cel-04 | 2400 | 8 | 195.2 | 36.8 |
| Cel-04 | 4800 | 5 | 202.0 | 68.4 |
| Cel-04 | 9600 | 7 | 219.5 | 32.3 |
| Hun-01 | 2400 | 8 | 181.8 | 38.3 |
| Hun-01 | 4800 | 7 | 220.9 | 27.4 |
| Hun-01 | 8000 | 6 | 217.3 | 33.2 |
| Hun-02 | 12000 | 7 | 218.5 | 43.6 |

In the protocols Hun-01 and Hun-02 within the 4800 IU/mL dose group two patients had baseline cholesterol levels in the 300s. The patient population had high rates of smoking and alcohol consumption (activities known to be associated with Head and Neck cancer and increased risk of liver disease) prior to and during the LI therapy. This was not readily apparent in other protocol populations. The Cel-01 trial was the only trial which included patients with recurring and Metastatic cancer.

Methods

Statistical meta-analysis was conducted on data collected in studies Cel-04 (50234-01), Hun-01 (50234-03) and Cel-01 (33575-01) using PC SAS 9.0. Data for protocols Cel-04, Cel-01 and Hun-01 (1st 39 patients) were extracted from preexisting SAS or Excel files. The data from protocol Hun-02 (50234-03) (2nd 20 patients) were imported from a text file supplied by a Contract Research Organization (CRO). In these data, the zero values were replaced by missing values (per CRO export error). Data for patients which did not include baseline or day 1 values or had a singular data point for cholesterol, were not included in the aggregate cholesterol dataset. Cholesterol data values were converted from mmol/L to mg/dL using the conversion constant of 38.67. The conversion was conducted to provide a more familiar context for the review of results.

Doses, injection frequency and site of injection varied between and within protocols. There was no "control" for these sources of variation in the model and means because of the sample sizes within each protocol and the subsequent loss of power within models. The lack of accounting for these factors predominantly reduces the statistical significance of the results. However, as will be seen in the tables and examples below, the results remain significant regardless of these confounders.

Day numbers are assigned with day one defined as the first day of treatment. The day numbers in these analyses reflect the elapsed number of days since the first day of treatment, and not actual treatment days. Thus, some patients had completed the trial by day 18, yet remained in follow, or did not. Other patients had additional or extended courses of treatment past day 18.

To more accurately model the estimated effects of IL-2 injection, the "within subject change values" of cholesterol were used as an outcome measure in ANOVA endpoint analysis and regression. These change values were defined in two forms for the MANOVA model:

where baseline = average (of screen & day1) Day18* = average (of day 18, 20 & 28).

$$Endpt1 = \frac{(Baseline - Day\ 18^{*})}{Baseline}$$

where baseline = average (of screen & day1) & Day35* = average (of day 35 & 40).

$$Endpt2 = \frac{(Baseline - Day\ 35^{*})}{Baseline}$$

Regression and Graphic display of Entire Pooled Database

The meta-data take on-the-whole shows one general picture, as represented in the Table 3 displayed below. This graphic representation depicts a clear downward trend in the cholesterol concentration values over time. The regression model of this plot is presented below. The slope is of these data regressed over days, is -0.64.

## Table 3

### Distribution of individual cholesterol concentrations by test date, Data are include all data retained for analysis from protocols 50234-01, 50234-02 and 50234-03 and 33575-01

```
Cholesterol Concentration

       350 ⌐
           ,
           ,   A
           ,                                         A
           ,
           ;  AA
       300 ┴
           ,   A            A
           ,  DB       A                             A
chol_level ;   A        A                            A
           ,  CB        D
           ,  A         D                            A
           ┴ ED        A    G    A
       250   EG        A    B                   A
           , HF        A    B              A
           , AA        B    CA         A
           , FG             G          A
           , HI             DA         A            A
           , MJ        A    D    A    A B        A
           , FI        C    G         A    A      A
       200 ┴ IH        C    HA   A          A
           , FH        C    KA   D    A    A    A
           , FH        B    LB        A    A    A
           , HS        B    GB   A    D
           , GJ        D    JA        C    A    A    A
           , FD        B    EC   D    B    C        B    A    A
           , EH        A    IA   A    D    A    B        A
       150 ┴ BA        A    F    A         A              A
           , .AA       A    B    A    A    A    A    B
           , AA        A    A    A         B         A
           ,  B             A
           ,  A                 A    A         A
           ,  B        A    A    A
       100 ┴                         A    A

           ┴──────────┴─────────┴─────────┴─────────┴──────────
           0         20        40        60        80
                                                    Day
```

Regression Model

The regression model is statistically, highly significant, p< 0.0001. Both the durational effect of therapy as well as the dose of therapy contributed to the reduction in average cholesterol concentrations. The estimate slope of change due to each additional day is -0.641 as shown in Table 4. This indicates that for each day of treatment the average level of cholesterol (across all groups) decreased by -0.64. This estimate is statistically significant from zero, signifying that there is a true reduction due to treatment. Given the observational (and purposively selected) nature of the data, this equation cannot be used to make inferences to the general population. However, the data strongly suggests a relationship exists between Leukocyte Interleukin injection (LI) treatment and serum cholesterol change.

### Table 4
### Regression Model of Cholesterol values aggregated across different labs & protocols, against Treatment length and Dose level.

The REG Procedure Model: MODEL1 Dependent Variable: chol_level

| Source | DF | Sum of Squares | Mean Square | F Value | Pr>F |
|--------|----|----|----|----|----|
| Model | 2 | 80274 | 40137 | 28.72 | <0.0001 |
| Error | 487 | 680534 | 1397.39986 | | |

(continued)

Regression Model of Cholesterol values aggregated across different labs & protocols, against Treatment length and Dose level.

The REG Procedure Model: MODEL1 Dependent Variable: chol_level

| Source | DF | Sum of Squares | Mean Square | F Value | Pr>F |
|---|---|---|---|---|---|
| Corrected Total | 489 | 760808 | | | |

| | Root MSE | | 37.38181 | R-Square | 0.1055 |
|---|---|---|---|---|---|
| | Dependent Mean | | 198.01486 | Adj R-Sq | 0.1018 |
| | Coeff Var | | 18.87829 | | |

Parameter Estimates

| Variable | Label | DF | Parameter Estimate | Standard Error | t Value | Pr> |t| |
|---|---|---|---|---|---|---|
| Intercept | Intercept | 1 | 198.13752 | 2.70045 | 73.37 | <0.0001 |
| Day | Day | 1 | -0.64092 | 0.11254 | -5.70 | <0.0001 |
| total_dose | total dose | 1 | 0.00220 | 0.00044071 | 4.99 | <0.0001 |

As seen in the data in Table 5 the simultaneous effects (Type III sum of squares), of duration (day) and the crossed effect of protocol and dose, the latter remain significant and our model the $R^2$ (amount of explained variance) increases to 27%, with a model p<0.0001. The cross effect means that there was a slightly different effect in studies even when they used the same dose of Leukocyte Interleukin Injection (LI) treatment.

Table 5

GLM Procedure Dependent Variable: chol_level

| Source | DF | Sum of Squares | Mean Square | F Value | Pr>F |
|---|---|---|---|---|---|
| Model | 31 | 206096.3973 | 6648.2709 | 5.49 | 0.0001 |
| Error | 458 | 554711.2664 | 1211.1600 | | |
| Corrected Total | 489 | 760807.6637 | | | |

| R-Square | Coeff Var | Root MSE | chol_level Mean |
|---|---|---|---|
| 0.270892 | 17.57531 | 34.80172 | 198.0149 |

| Source | DF | Type III SS | Mean Square | F Value | Pr>F |
|---|---|---|---|---|---|
| Day | 14 | 16624.9584 | 1187.4970 | 0.98 | 0.4722 |
| total_ dose*protocol | 17 | 138518.6574 | 8148.1563 | 6.73 | <0.0001 |

The relative change from baseline as a function of a patient's cholesterol levels at baseline and the dose received during treatment was also analyzed as shown in Table 6. The GLM model results indicated that there was a significant difference due to baseline cholesterol levels. Unfortunately, it is not possible to determine whether there is a different effect for patients with high or low relative levels of blood lipids or if the factor is a proxy for our study populations in each protocol.

## Table 6

### Average amount of Reduction in Cholesterol
### From baseline to Day 18
### Measured in mg/dl, by Dose group

| dose | N | Mean | Lower 95% CL for Mean | Upper 95% CL for Mean | Median |
|---|---|---|---|---|---|
| 200 | 5 | 4.1 | -9.0 | 17.1 | 3.9 |
| 400 | 3 | 6.6 | -16.4 | 29.7 | 2.9 |
| 800 | 13 | 0.3 | -10.3 | 10.9 | 4.3 |
| 1200 | 6 | 12.4 | -18.0 | 42.8 | 2.5 |
| 1600 | 9 | 10.4 | -5.9 | 26.7 | 11.4 |
| 2400 | 16 | -7.4 | -23.5 | 8.7 | -13.0 |
| 3200 | 12 | 15.9 | -0.4 | 32.2 | 18.1 |
| 4800 | 12 | 9.8 | -0.5 | 20.2 | 16.3 |
| 8000 | 6 | 3.6 | -29.9 | 37.1 | 3.8 |
| 9600 | 7 | 4.4 | -27.1 | 35.8 | 8.5 |
| 12000 | 3 | 35.7 | -23.2 | 94.6 | 41.4 |

endpt

| Source | DF | Sum of Squares | Mean Square | F Value | Pr > F |
|---|---|---|---|---|---|
| Model | 7 | 18.11382251 | 2.58768893 | 3.65 | 0.0098 |
| Error | 21 | 14.87077231 | 0.70813201 | | |
| Corrected Total | 28 | 32.98459483 | | | |

| R-Square | Coeff Var | Root MSE | endpt Mean |
|---|---|---|---|
| 0.549160 | 17.44552 | 0.841506 | 4.823621 |

| Source | DF | Type I SS | Mean Square | F Value | Pr > F |
|---|---|---|---|---|---|
| baseline | 1 | 11.56487006 | 11.56487006 | 16.33 | 0.0006 |
| dose | 6 | 6.54895246 | 1.09149208 | 1.54 | 0.2136 |

Mean Concentrations of Serum Cholesterol stratified by Protocol and Dose groups

Average cholesterol concentrations, for specific tests days, were stratified into dose groups: 200, 400, 800, 1200, 1600, 2400, 3200, 4800, 8000, 9600, and 12000 IU/mL. Several strata had very small number of observation (n), and consequently the confidence intervals about the mean were wide. With the small n, it is difficult to demonstrate significant differences between daily averages. However, the point estimates (means) clearly demonstrate a decrease in average cholesterol concentration over time in several of the dose groups.

Both the 1200 and 1600 IU/mL dose group means indicate a change from baseline. In the 1600 IU/mL dose group, an inconsistent number of observations per day are present. However, the point estimates (means) are supported by the concurrent median values. Both indicate a reduced level of cholesterol values over time.

In dose groups 2400, 3200 and 4800 IU/mL, there is a small shift in the point estimate. Only in the 3200 IU/mL group does the median and general shape of the cholesterol data distribution support the trend implied by the mean values. The means for dose group 4800 IU/mL demonstrate a small change from day 1 to day 18; however, given that n is 14, a statistical power was not provided to prove whether this change is statistically significant.

As is shown by the following examples, dose levels 1200, 1600 and 3200 IU/mL seem to have the strongest effect. Dose level 2400 IU/mL had several outliers which skewed the distribution and affected the mean estimate. Moreover, the mean estimates for ALT and AST did not shift from baseline to post treatment as shown in following examples. Most notably, none of the changes from baseline to post treatment for ALT and AST are statistically significant in any of the protocol populations suggesting LI's use for patients having compromised liver function.

Example 1

The following is results of a daily mean laboratory values for cholesterol aggregated across different labs & protocols, grouped by dose the dose level at 200 IU/mL as shown in Table 7.

Table 7

Dose level 200 IU/mL, Analysis Variable : chol_level

| Day | Obs | N Mean | Lower 95% CL for Mean | Upper 95% CL for Mean | Median |
|---|---|---|---|---|---|
| 0 | 5 | 192.5 | 146.0 | 239.0 | 196.1 |
| 1 | 5 | 184.1 | 144.1 | 224.0 | 181.0 |
| 10 | 5 | 185.2 | 145.1 | 225.3 | 183.7 |
| 18 | 5 | 183.2 | 143.5 | 222.9 | 178.7 |
| 20 | 5 | 183.5 | 152.6 | 214.3 | 174.8 |
| 28 | 5 | 170.4 | 138.3 | 202.5 | 179.0 |
| 35 | 5 | 171.1 | 122.3 | 220.0 | 165.3 |

Example 2

The following is results of a daily mean laboratory values for cholesterol aggregated across different labs & protocols, grouped by dose the dose level at 400 IU/mL as shown in Table 8.

Table 8

Dose level 400 IU/mL, Analysis Variable : chol_level

| Day | Obs | N Mean | Lower 95% CL for Mean | Upper 95% CL for Mean | Median |
|---|---|---|---|---|---|
| 0 | 3 | 190.3 | 138.7 | 241.8 | 188.3 |
| 1 | 3 | 203.0 | 153.6 | 252.4 | 208.4 |
| 10 | 3 | 192.1 | 144.4 | 239.8 | 195.7 |
| 18 | 2 | 181.2 | 95.2 | 267.2 | 181.2 |
| 20 | 3 | 187.2 | 155.3 | 219.0 | 183.3 |
| 28 | 3 | 175.6 | 108.9 | 242.2 | 189.5 |
| 35 | 3 | 166.2 | 152.2 | 180.1 | 168.6 |
| 40 | 2 | 160.7 | 158.2 | 163.1 | 160.7 |

Example 3

The following is results of a daily mean laboratory values for cholesterol aggregated across different labs & protocols, grouped by dose the dose level at 800 IU/mL as shown in Table 9.

Table 9

Dose level 800 IU/mL Analysis Variable: chol_level

| Day | Obs | N Mean | Lower 95% CL for Mean | Upper 95% CL for Mean | Median |
|---|---|---|---|---|---|
| 0 | 26 | 199.4 | 183.5 | 215.3 | 200.7 |
| 1 | 25 | 202.4 | 186.1 | 218.8 | 182.3 |
| 10 | 4 | 162.0 | 102.2 | 221.8 | 167.2 |
| 18 | 26 | 206.9 | 189.5 | 224.4 | 186.0 |
| 20 | 3 | 197.7 | 121.3 | 274.2 | 185.2 |
| 28 | 3 | 176.2 | 120.8 | 231.6 | 165.5 |
| 35 | 3 | 175.3 | 156.2 | 194.4 | 177.9 |

Example 4

The following is results of a daily mean laboratory values for cholesterol aggregated across different labs & protocols, grouped by dose the dose level at 1200 IU/mL as shown in Table 10.

Table 10

Dose level 1200 IU/mL, Analysis Variable : chol_level

| Day | Obs | N Mean | Lower 95% CL for Mean | Upper 95% CL for Mean | Median |
|---|---|---|---|---|---|
| 0 | 6 | 237.1 | 193.3 | 280.9 | 242.5 |
| 1 | 6 | 218.5 | 167.4 | 269.7 | 209.4 |
| 18 | 6 | 215.4 | 178.3 | 252.5 | 208.2 |

Example 5

The following is results of a daily mean laboratory values for cholesterol aggregated across different labs & protocols, grouped by dose the dose level at 1600 IU/mL as shown in Table 11.

Table 11

Dose level 1600 IU/mL, Analysis Variable: chol_level

| Day | Obs | N Mean | Lower 95% CL for Mean | Upper 95% CL for Mean | Median |
|---|---|---|---|---|---|
| 0 | 20 | 207.7 | 186.7 | 228.6 | 213.3 |
| 1 | 20 | 208.9 | 192.4 | 225.4 | 198.4 |
| 10 | 4 | 149.4 | 103.0 | 195.8 | 139.6 |
| 18 | 20 | 195.2 | 175.2 | 215.2 | 188.7 |
| 20 | 4 | 149.8 | 97.1 | 202.5 | 161.3 |
| 28 | 4 | 137.9 | 98.5 | 177.2 | 137.5 |
| 35 | 4 | 138.1 | 97.2 | 178.9 | 140.2 |
| 40 | 3 | 136.6 | 67.4 | 205.9 | 138.4 |

Example 6

The following is results of a daily mean laboratory values for cholesterol aggregated across different labs & protocols, grouped by dose the dose level at 2400 IU/mL as shown in Table 12.

Table 12

Dose level 1600 IU/mL, Analysis Variable : chol_level

| Day | Obs | N Mean | Lower 95% CL for Mean | Upper 95% CL for Mean | Median |
|---|---|---|---|---|---|
| 0 | 11 | 201.1 | 175.6 | 226.6 | 204.2 |
| 1 | 17 | 180.6 | 161.1 | 200.0 | 179.8 |
| 18 | 16 | 195.9 | 176.9 | 214.9 | 202.8 |
| 35 | 2 | 164.5 | 88.4 | 240.7 | 164.5 |

Example 7

The following is results of a daily mean laboratory values for cholesterol aggregated across different labs & protocols, grouped by dose the dose level at 3200 IU/mL as shown in Table 13.

Table 13

Dose level 3200 IU/mL, Analysis Variable : chol_level

| Day | Obs | N Mean | Lower 95% CL for Mean | Upper 95% CL for Mean | Median |
|---|---|---|---|---|---|
| 0 | 18 | 205.7 | 194.2 | 217.1 | 210.8 |
| 1 | 18 | 200.8 | 186.4 | 215.2 | 206.9 |
| 18 | 17 | 188.0 | 175.6 | 200.4 | 195.3 |

Example 8

The following is results of a daily mean laboratory values for cholesterol aggregated across different labs & protocols, grouped by dose the dose level at 4800 IU/mL as shown in Table 14.

Table 14

Dose level 4800 IU/mL, Analysis Variable : chol_level

| Day | Obs | N Mean | Lower 95% CL for Mean | Upper 95% CL for Mean | Median |
|---|---|---|---|---|---|
| 0 | 15 | 207.9 | 186.8 | 228.9 | 216.2 |
| 1 | 12 | 199.2 | 177.0 | 221.4 | 202.1 |
| 18 | 13 | 197.0 | 174.8 | 219.2 | 194.5 |

Example 9

The following is results of a daily mean laboratory values for cholesterol aggregated across different labs & protocols, grouped by dose the dose level at 8000 IU/mL as shown in Table 15.

Table 15

Day level 8000 IU/mL, Analysis Variable: chol_level

| Day | Obs | N Mean | Lower 95% CL for Mean | Upper 95% CL for Mean | Median |
|---|---|---|---|---|---|
| 0 | 5 | 209.9 | 158.7 | 261.1 | 203.0 |
| 1 | 5 | 214.5 | 162.6 | 266.5 | 213.1 |

(continued)

Day level 8000 IU/mL, Analysis Variable: chol_level

| Day | Obs | N Mean | Lower 95% CL for Mean | Upper 95% CL for Mean | Median |
|---|---|---|---|---|---|
| 18 | 6 | 213.7 | 161.1 | 266.2 | 210.8 |

Example 10

The following is results of a daily mean laboratory values for cholesterol aggregated across different labs & protocols, grouped by dose the dose level at 9600 IU/mL as shown in Table 16.

Table 16

Dose level 9600 IU/mL, Analysis Variable : chol_level

| Day | Obs | N Mean | Lower 95% CL for Mean | Upper 95% CL for Mean | Median |
|---|---|---|---|---|---|
| 0 | 7 | 223.1 | 189.1 | 257.0 | 222.7 |
| 1 | 7 | 215.9 | 189.7 | 242.1 | 223.9 |
| 18 | 7 | 215.1 | 177.4 | 252.8 | 226.2 |

Example 11

The following is results of a daily mean laboratory values for cholesterol aggregated across different labs & protocols, grouped by dose the dose level at 12000 IU/mL as shown in Table 17.

Table 17

Dose level 12000 IU/mL, Analysis Variable: chol_level

| Day | Obs | N Mean | Lower 95% CL for Mean | Upper 95% CL for Mean | Median |
|---|---|---|---|---|---|
| 1 | 22 | 221.2 | 201.0 | 241.4 | 215.0 |
| 10 | 17 | 202.3 | 182.7 | 222.0 | 208.8 |
| 18 | 2 | 181.0 | 87.6 | 274.3 | 181.0 |
| 28 | 3 | 235.5 | -54.4 | 525.4 | 235.5 |
| 35 | 7 | 202.5 | 176.6 | 228.4 | 193.4 |
| 40 | 13 | 232.4 | 192.1 | 272.8 | 216.6 |

Example 12

The following is results of ALT values showing no statistically significant change from baseline to post treatment for patients in protocol Cel-02 (50234-02) as shown in Table 18.

Table 18

Alanine Transaminase (ALT) The MEANS Procedure Analysis Variable : Lab_Value Lab Value

| Day | Obs | N Mean | Std Dev | Median |
|---|---|---|---|---|
| Screen | 33 | 23.5 | 10.4 | 22.0 |
| Day 1 | 33 | 22.1 | 10.5 | 19.0 |
| Post | 33 | 28.1 | 21.8 | 23.0 |

Example 13

The following is results of AST values showing no statistically significant change from baseline to post treatment for patients in protocol Cel-02 (50234-02) as shown in Table 19.

Table 19
Aspartate Transaminase (AST)
Analysis Variable : Lab_Value Lab Value

| Day | Obs | N Mean | Std Dev | Median |
|---|---|---|---|---|
| Screen | 33 | 25.9 | 7.2 | 26.0 |
| Day 1 | 33 | 24.3 | 6.6 | 24.0 |
| Post | 33 | 27.2 | 18.0 | 22.0 |

Example 14

The following is results of ALT values showing no statistically significant change from baseline to post treatment for patients in protocol Cel-01 (33575-01) as shown in Table 20.

Table 20
Alanine Transaminase (ALT)
The MEANS Procedure
Analysis Variable : Lab_Value Lab Value

| Day | Obs | N Mean | Std Dev | Median |
|---|---|---|---|---|
| screen | 3 | 16.0 | 2.6 | 17.0 |
| C1D1 | 3 | 14.3 | 2.3 | 13.0 |
| C1D12 | 5 | 16.4 | 2.1 | 17.0 |
| C1D5 | 3 | 13.7 | 1.2 | 13.0 |
| C2D1 | 4 | 15.3 | 3.1 | 16.0 |
| C2D15 | 2 | 13.5 | 0.7 | 13.5 |
| C2D8 | 2 | 14.5 | 0.7 | 14.5 |
| C3D1 | 6 | 15.2 | 2.7 | 15.5 |

C = Treatment Cycle
D = Day
e.g. C1D1 = Treatment Cycle 1, Day 1

Example 15

The following is results of AST values showing no statistically significant change from baseline to post treatment for patients in protocol Cel-01 (33575-01) as shown in Table 21.

Table 21
Aspartate Transaminase (AST)
Analysis Variable : Lab_Value Lab Value

| Day | Obs | N Mean | Std Dev | Median |
|---|---|---|---|---|
| screen | 16 | 23.2 | 6.8 | 21.0 |
| C1D1 | 14 | 24.7 | 10.1 | 22.0 |
| C1D12 | 14 | 24.6 | 8.8 | 24.5 |

(continued)

Aspartate Transaminase (AST)

Analysis Variable : Lab_Value Lab Value

| Day | Obs | N Mean | Std Dev | Median |
|---|---|---|---|---|
| C1D5 | 15 | 24.9 | 9.5 | 25.0 |

C = Treatment Cycle
D = Day
e.g. C1D1 = Treatment Cycle 1, Day 1

Example 16

The following is results of AST values showing no statistically significant change from baseline to post treatment for patients in protocol Cel-01 (33575-01) as shown in Table 22.

Table 22

Aspartate Transaminase (AST)

The MEANS Procedure

Analysis Variable : Lab_Value Lab Value

| Day | Obs | N Mean | Std Dev | Median |
|---|---|---|---|---|
| C2D1 | 14 | 23.3 | 9.7 | 21.5 |
| C2D15 | 14 | 29.2 | 36.8 | 19.0 |
| C2D8 | 15 | 22.2 | 8.8 | 20.0 |
| C3D1 | 11 | 24.4 | 13.6 | 21.0 |
| C4D1 | 4 | 25.5 | 18.4 | 17.0 |
| C5D1 | 3 | 36.0 | 32.1 | 20.0 |

C = Treatment Cycle
D = Day
e.g. C1D1 = Treatment Cycle 1, Day I

Example 17

The following is results of ALT values showing no statistically significant change from baseline to post treatment for patients in protocol Cel-04 (50234-01) as shown in Table 23.

Table 23

Alanine Transaminase (ALT)

Analysis Variable : Lab_Value Lab Value

| Day | Obs | N Mean | Std Dev | Median |
|---|---|---|---|---|
| Screen | 26 | 26.6 | 12.7 | 23.5 |
| Day 1 | 26 | 24.7 | 13.0 | 21.0 |
| Post | 26 | 28.8 | 11.2 | 26.0 |

Example 18

The following is results of AST values showing no statistically significant change from baseline to post treatment for patients in protocol Cel-04 (50234-01) as shown in Table 24.

Table 24
Aspartate Transaminase (AST)
The MEANS Procedure

Analysis Variable : Lab_Value Lab Value

| Day | Obs | N Mean | Std Dev | Median |
|---|---|---|---|---|
| Screen | 26 | 28.5 | 17.9 | 21.0 |
| Day 1 | 26 | 32.6 | 27.2 | 21.0 |
| Post | 26 | 25.9 | 9.3 | 25.0 |

Example 19

The following is results of AST values showing no statistically significant change from baseline to post treatment for patients in protocol Hun-01 (50234-03) as shown in Table 25.

Table 25
Aspartate Transaminase (AST)
Analysis Variable : Lab_Value Lab Value

| Day | Obs | N Mean | Std Dev | Median |
|---|---|---|---|---|
| Screen | 21 | 27.8 | 15.5 | 22.0 |
| Day 1 | 29 | 23.4 | 17.5 | 18.0 |
| Post | 29 | 21.4 | 8.4 | 19.0 |
| z- follow up | 8 | 19.3 | 2.3 | 20.0 |

Example 20

The following is results of ALT values showing no statistically significant change from baseline to post treatment for patients in protocol Hun-02 (50234-03) as shown in Table 26.

Table 26
Alanine Transaminase (ALT)
The MEANS Procedure

Analysis Variable : Lab_Value Lab Value

| Day | Obs | N Mean | Std Dev | Median |
|---|---|---|---|---|
| Screen | 15 | 25.4 | 22.8 | 16.2 |
| Day 1 | 20 | 28.7 | 37.4 | 15.5 |
| Post | 20 | 22.8 | 17.3 | 16.0 |
| z- follow up | 14 | 43.6 | 32.0 | 29.0 |

Example 21

The following is results of AST values showing no statistically significant change from baseline to post treatment for patients in protocol Hun-02 (50234-03) as shown in Table 27.

EP 1 773 395 B1

Table 27
Aspartate TransaEninase (AST)
Analysis Variable : Lab_Vatue Lab Value

| Day | N Obs | Mean | Std Dev | Median |
|---|---|---|---|---|
| a-screen | 15 | 31.9 | 27.4 | 25.0 |
| Day 1 | 20 | 41.2 | 54.4 | 17.5 |
| Post | 20 | 26.5 | 22.1 | 20.4 |
| z- followup | 14 | 43.3 | 34.8 | 24.0 |

The invention being thus described, it will be obvious that the same may be varied in many ways. Such variations are not to be regarded as a departure from the spirit scope of the invention and all such modifications are intended to be included within the scope of the following claims.

**Claims**

1. A serum-free and mitogen-free cytokine mixture comprised of specific ratios of cytokines selected from the group of IL-1β, TNF-α, IFN-γ and GM-CSF to Interleukin-2 (IL-2):

   IL-1β to IL-2 at a ratio range of 0.4 - 1.5;
   TNF-α to IL-2 at a ratio range of 3.2 - 11.3;
   IFN-γ to IL-2 at a ratio range of 1.5 - 10.9; and
   GM-CSF to IL-2 at a ratio range of 2.2 - 4.8 for use in treating or preventing a disease or disorder that is capable of being treated or prevented by lowering cholesterol levels.

2. The serum-free and mitogen-free cytokine mixture of claim 1, wherein the specific ratios of cytokines are:

   IL-1β to IL-2 at a ratio range of 0.6 to 0.8;
   TNF-α to IL-2 at a ratio range of 7.7 to 10.9;
   IFN-γ to IL-2 at a ratio range of 4.9 to 7.1; and
   GM-CSF to IL-2 at a ratio range of 3.5 to 4.5.

3. The serum-free and mitogen-free cytokine mixture of claim 1, wherein said serum-free and mitogen-free cytokine mixture is administered three times a week over a two week period in a range from about 20 IU to 1600 IU wherein IU represent International Units for Interleukin-2 given in World Health Organization 1st International Standard for Human IL-2, 86/504.

4. The serum-free and mitogen-free cytokine mixture of claim 3, wherein said serum-free and mitogen-free cytokine mixture is administered three times a week over a two week period in a range from about 40 IU to 800 wherein IU represent International Units for Interleukin-2 given in World Health Organization 1st International Standard for Human IL-2, 86/504.

5. The serum-free and mitogen-free cytokine mixture of claim 4, wherein said serum-free and mitogen-free cytokine mixture is administered three times a week over a two week period in a range from about 35 IU to 75 IU wherein IU represent International Units for Interleukin-2 given in World Health Organization 1st International Standard for Human IL-2, 86/504.

6. The serum-free and mitogen-free cytokine mixture of claim 1, wherein said serum-free and mitogen-free cytokine mixture is administered three times a week over a two week period at 55 IU wherein IU represent International Units for Interleukin-2 given in World Health Organization 1st International Standard for Human IL-2, 86/504.

7. The serum-free and mitogen-free cytokine mixture of claim 1, wherein said serum-free and mitogen-free cytokine mixture is administered three times a week over a two week period at 200 IU wherein IU represent International Units for Interleukin-2 given in World Health Organization 1st International Standard for Human IL-2, 86/504.

8. The serum-free and mitogen-free cytokine mixture of claim 1, wherein said serum-free and mitogen-free cytokine mixture is administered three times a week over a two week period at 400 IU wherein IU represent International Units for Interleukin-2 given in World Health Organization 1st International Standard for Human IL-2, 86/504.

9. The serum-free and mitogen-free cytokine mixture of claim 1, wherein said serum-free and mitogen-free cytokine mixture is administered three times a week over a two week period at 800 IU wherein IU represent International Units for Interleukin-2 given in World Health Organization 1st International Standard for Human IL-2, 86/504.

10. The serum-free and mitogen-free cytokine mixture of claim 1, wherein said serum-free and mitogen-free cytokine mixture is administered three times a week over a two week period at 1200 IU wherein IU represent International Units for Interleukin-2 given in World Health Organization 1st International Standard for Human IL-2, 86/504.

11. The serum-free and mitogen-free cytokine mixture of claim 1, wherein said serum-free and mitogen-free cytokine mixture is administered three times a week over a two week period at 1600 IU wherein IU represent International Units for Interleukin-2 given in World Health Organization 1st International Standard for Human IL-2, 86/504.

12. The serum-free and mitogen-free cytokine mixture of claim 1, wherein said serum-free and mitogen-free cytokine mixture is administered three times a week over a two week period at 2400 IU wherein IU represent International Units for Interleukin-2 given in World Health Organization 1st International Standard for Human IL-2, 86/ 504.

13. The serum-free and mitogen-free cytokine mixture of claim 1, wherein said serum-free and mitogen-free cytokine mixture is administered three times a week over a two week period at 3200 IU wherein IU represent International Units for Interleukin-2 given in World Health Organization 1st International Standard for Human IL-2, 86/504.

14. The serum-free and mitogen-free cytokine mixture of claim 1, wherein said serum-free and mitogen-free cytokine mixture is administered three times a week over a two week period at 4800 IU wherein IU represent International Units for Interleukin-2 given in World Health Organization 1st International Standard for Human IL-2, 86/504.

15. The serum-free and mitogen-free cytokine mixture of claim 1, wherein said serum-free and mitogen-free cytokine mixture is administered three times a week over a two week period at 8000 IU wherein IU represent International Units for Interleukin-2 given in World Health Organization 1st International Standard for Human IL-2, 86/504.

16. The serum-free and mitogen-free cytokine mixture of claim 1, wherein said serum-free and mitogen-free cytokine mixture is administered three times a week over a two week period at 9600 IU wherein IU represent International Units for Interleukin-2 given in World Health Organization 1st International Standard for Human IL-2, 86/504.

17. The serum-free and mitogen-free cytokine mixture of claim 1, wherein said serum-free and mitogen-free cytokine mixture is administered three times a week over a two week period at 12000 IU wherein IU represent International Units for Interleukin-2 given in World Health Organization 1st International Standard for Human IL-2, 86/504.

18. The serum-free and mitogen-free cytokine mixture of claim 1, wherein said serum-free and mitogen-free cytokine mixture is administered five times a week over a three week period at 200 IU wherein IU represent International Units for Interleukin-2 given in World Health Organization 1st International Standard for Human IL-2, 86/504.

19. The serum-free and mitogen-free cytokine mixture of claim 1, wherein said serum-free and mitogen-free cytokine mixture is administered five times a week over a three week period at 400 IU wherein IU represent International Units for Interleukin-2 given in World Health Organization 1st International Standard for Human IL-2, 86/504.

20. The serum-free and mitogen-free cytokine mixture of claim 1, wherein said serum-free and mitogen-free cytokine mixture is administered five times a week over a three week period at 800 IU wherein IU represent International Units for Interleukin-2 given in World Health Organization 1st International Standard for Human IL-2, 86/504.

21. The serum-free and mitogen-free cytokine mixture of claim 1, wherein said serum-free and mitogen-free cytokine mixture is administered five times a week over a three week period at 1200 IU wherein IU represent International Units for Interleukin-2 given in World Health Organization 1st International Standard for Human IL-2, 86/504.

22. The serum-free and mitogen-free cytokine mixture of claim 1, wherein said serum-free and mitogen-free cytokine mixture is administered five times a week over a three week period at 1600 IU wherein IU represent International Units for Interleukin-2 given in World Health Organization 1st International Standard for Human IL-2, 86/504.

23. The serum-free and mitogen-free cytokine mixture of claim 1, wherein said serum-free and mitogen-free cytokine mixture is administered five times a week over a three week period at 2400 IU wherein IU represent International Units for Interleukin-2 given in World Health Organization 1st International Standard for Human IL-2, 86/504.

24. The serum-free and mitogen-free cytokine mixture of claim 1, wherein said serum-free and mitogen-free cytokine mixture is administered five times a week over a three week period at 3200 IU wherein IU represent International Units for Interleukin-2 given in World Health Organization 1st International Standard for Human IL-2, 86/504.

25. The serum-free and mitogen-free cytokine mixture of claim 1, wherein said serum-free and mitogen-free cytokine mixture is administered five times a week over a three week period at 4800 IU wherein IU represent International Units for Interleukin-2 given in World Health Organization 1st International Standard for Human IL-2, 86/504.

26. The serum-free and mitogen-free cytokine mixture of claim 1, wherein said serum-free and mitogen-free cytokine mixture is administered five times a week over a three week period at 8000 IU wherein IU represent International Units for Interleukin-2 given in World Health Organization 1st International Standard for Human IL-2, 86/504.

27. The serum-free and mitogen-free cytokine mixture of claim 1, wherein said serum-free and mitogen-free cytokine mixture is administered five times a week over a three week period at 9600 IU wherein IU represent International Units for Interleukin-2 given in World Health Organization 1st International Standard for Human IL-2, 86/504.

28. The serum-free and mitogen-free cytokine mixture of claim 1, wherein said serum-free and mitogen-free cytokine mixture is administered five times a week over a three week period at 12000 IU wherein IU represent International Units for Interleukin-2 given in World Health Organization 1st International Standard for Human IL-2, 86/504.

29. The serum-free and mitogen-free cytokine mixture of claim 1, wherein said serum-free and mitogen-free cytokine mixture is administered three times a week over an eight to twenty-six week period in a range from about 20 IU to 1600 IU wherein IU represent International Units for Interleukin-2 given in World Health Organization 1st International Standard for Human IL-2, 86/ 504.

30. The serum-free and mitogen-free cytokine mixture of claim 3, wherein said serum-free and mitogen-free cytokine mixture is administered five times a week over an eight to twenty-six week period in a range from about 20 IU to 1600 IU wherein IU represent International Units for Interleukin-2 given in World Health Organization 1st International Standard for Human IL-2, 86/504.

31. The serum-free and mitogen-free cytokine mixture of claim 1, wherein the disease or disorder is selected from the group consisting of cardiovascular disease, dyslipidemia, dyslipidproteinemia, hypertension, and hyperlipidemia.

**Patentansprüche**

1. Eine Serum-freie und Mitogen-freie Zytokinmischung, gebildet aus spezifischen Verhältniswerten von Zytokinen ausgewählt aus der Gruppe bestehend aus IL-1$\beta$, TNF-$\alpha$, IFN-$\gamma$ and GM-CSF zu Interleukin-2 (IL-2):

   IL-1$\beta$ zu IL-2 in einem Verhältniswertebereich von 0,4 - 1,5;
   TNF-$\alpha$ zu IL-2 in einem Verhältniswertebereich von 3,2 - 11,3;
   IFN-$\gamma$ zu IL-2 in einem Verhältniswertebereich von 1,5 - 10,9; und
   GM-CSF zu IL-2 in einem Verhältniswertebereich von 2,2 - 4,8 für die Verwendung im Behandeln oder Vorbeugen einer Krankheit oder Funktionsstörung, die durch das Senken von Cholesterol-Gehalten behandelt werden kann oder der durch das Senken von Cholesterol-Gehalten vorgebeugt werden kann.

2. Die Serum-freie und Mitogen-freie Zytokinmischung nach Anspruch 1, wobei die spezifischen Verhältniswerte der Zytokine sind:

   IL-1$\beta$ zu IL-2 in einem Verhältniswertebereich von 0,6 - 0,8;
   TNF-$\alpha$ zu IL-2 in einem Verhältniswertebereich von 7,7 - 10,9;
   IFN-$\gamma$ zu IL-2 in einem Verhältniswertebereich von 4,9 - 7,1; und

GM-CSF zu IL-2 in einem Verhältniswertebereich von 3,5 - 4,5.

**3.** Die Serum-freie und Mitogen-freie Zytokinmischung nach Anspruch 1, wobei die Serum-freie und Mitogen-freie Zytokinmischung drei Mal pro Woche über einen Zeitraum von zwei Wochen in einem Bereich von ungefähr 20 IU bis 1600 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**4.** Die Serum-freie und Mitogen-freie Zytokinmischung nach Anspruch 3, wobei die Serum-freie und Mitogen-freie Zytokinmischung drei Mal pro Woche über einen Zeitraum von zwei Wochen in einem Bereich von ungefähr 40 IU bis 800 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**5.** Die Serum-freie und Mitogen-freie Zytokinmischung nach Anspruch 4, wobei die Serum-freie und Mitogen-freie Zytokinmischung drei Mal pro Woche über einen Zeitraum von zwei Wochen in einem Bereich von ungefähr 35 IU bis 75 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**6.** Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 55 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**7.** Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 200 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**8.** Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 400 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**9.** Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 800 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**10.** Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 1200 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**11.** Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 1600 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**12.** Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 2400 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

13. Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 3200 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

14. Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 4800 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

15. Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 8000 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

16. Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 9600 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

17. Die Serum-freie und Mitogen-freie Zytokinmischung nach Anspruch 1, wobei die Serum-freie und Mitogen-freie Zytokinmischung drei Mal pro Woche über einen Zeitraum von zwei Wochen mit 12000 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

18. Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 200 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

19. Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 400 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

20. Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 800 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

21. Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 1200 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**22.** Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 1600 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**23.** Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 2400 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**24.** Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 3200 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**25.** Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 4800 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**26.** Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 8000 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**27.** Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 9600 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**28.** Die Serum-freie und Mitogen-freie Zytokinmischung nach Anspruch 1, wobei die Serum-freie und Mitogen-freie Zytokinmischung fünf Mal pro Woche über einen Zeitraum von drei Wochen mit 12000 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**29.** Die Serum-freie und Mitogen-freie Zytokinmischung nach Anspruch 1, wobei die Serum-freie und Mitogen-freie Zytokinmischung drei Mal pro Woche über einen Zeitraum von 8 bis 26 Wochen in einem Bereich von ungefähr 20 IU bis 1600 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**30.** Die Serum-freie und Mitogen-freie Zytokinmischung nach Anspruch 3, wobei die Serum-freie und Mitogen-freie Zytokinmischung fünf Mal pro Woche über einen Zeitraum von 8 bis 26 Wochen in einem Bereich von ungefähr 20 IU bis 1600 IU verabreicht wird, wobei IU die internationalen Einheiten (International Units) für Interleukin-2 repräsentiert, die im 1. Internationalen Standard für humanes IL-2 der Weltgesundheitsorganisation (World Health Organisation 1st International Standard for Human IL-2), 86/504, vorgegeben werden.

**31.** Die Serum-freie und Mitogen-freie Zytokinmischung nach Anspruch 1, wobei die Krankheit oder Funktionsstörung ausgewählt ist aus der Gruppe bestehend aus Herz-Kreislauf-Erkrankung, Dyslipidämie, Dyslipoproteinämie, Bluthochdruck und Hyperlipidämie.

**Revendications**

1. Mélange de cytokines exempt de sérum et exempt d'agents mitogènes, composé de rapports spécifiques de cytokines choisis dans le groupe de IL-1β, TNF-α, IFN-γ et GM-CSF à l'interleukine-2 (IL-2):

   IL-1β à IL-2 dans un intervalle de rapport de 0,4-1,5;
   TNF-α à IL-2 dans un intervalle de rapport de 3,2-11,3;
   IFN-γ à IL-2 dans un intervalle de rapport de 1,5-10,9; et
   GM-CSF à IL-2 dans un intervalle de rapport de 2,2-4,8 pour utilisation dans le traitement ou la prévention d'une maladie ou d'un trouble qui est apte à être traité ou prévenu par un abaissement des taux de cholestérol.

2. Mélange de cytokines exempt de sérum et exempt d'agents mitogènes selon la revendication 1, dans lequel les rapports spécifiques des cytokines sont:

   IL-1β à IL-2 dans un intervalle de rapport de 0,6 à 0,8;
   TNF-α à IL-2 dans un intervalle de rapport de 7,7 à 10,9;
   IFN-γ à IL-2 dans un intervalle de rapport de 4,9 à 7,1; et
   GM-CSF à IL-2 dans un intervalle de rapport de 3,5 à 4,5.

3. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines dans un intervalle d'environ 20 UI à 1600 UI, tandis que UI représente les unités internationales pour l'interleukine-2 indiquées dans la 1ère norme internationale pour la IL-2 humaine, 86/504 de l'organisation mondiale de la santé.

4. Mélange de cytokines exempt de sérum et exempt d'agents mitogènes selon la revendication 3, dans lequel ledit mélange de cytokines exempt de sérum et exempt d'agents mitogènes est administré trois fois par semaine sur une période de deux semaines dans un intervalle d'environ 40 UI à 800 UI, tandis que UI représente les unités internationales pour l'interleukine-2 indiquées dans la 1ère norme internationale pour la IL-2 humaine, 86/504 de l'organisation mondiale de la santé.

5. Mélange de cytokines exempt de sérum et exempt d'agents mitogènes selon la revendication 4, dans lequel ledit mélange de cytokines exempt de sérum et exempt d'agents mitogènes est administré trois fois par semaine sur une période de deux semaines dans un intervalle d'environ 35 UI à 75 UI, tandis que UI représente les unités internationales pour l'interleukine-2 indiquées dans la 1ère norme internationale pour la IL-2 humaine, 86/504 de l'organisation mondiale de la santé.

6. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 55 UI, tandis que UI représente les unités internationales pour l'interleukine-2 indiquées dans la 1ère norme internationale pour la IL-2 humaine, 86/504 de l'organisation mondiale de la santé.

7. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 200 UI, tandis que UI représente les unités internationales pour l'interleukine-2 indiquées dans la 1ère norme internationale pour la IL-2 humaine, 86/504 de l'organisation mondiale de la santé.

8. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 400 UI, tandis que UI représente les unités internationales pour l'interleukine-2 indiquées dans la 1ère norme internationale pour la IL-2 humaine, 86/504 de l'organisation mondiale de la santé.

9. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 800 UI, tandis que UI représente les unités internationales pour l'interleukine-2 indiquées dans la 1<sup>ére</sup> norme internationale pour la IL-2 humaine, 86/504 de l'organisation mondiale de la santé.

10. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 1200 UI, tandis que UI représente les unités internationales pour l'interleukine-2 indiquées dans la 1<sup>ère</sup> norme internationale pour la IL-2 humaine, 86/504 de l'organisation mondiale de la santé.

11. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 1600 UI, tandis que UI représente les unités internationales pour l'interleukine-2 indiquées dans la 1<sup>ère</sup> norme internationale pour la IL-2 humaine, 86/504 de l'organisation mondiale de la santé.

12. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 2400 UI, tandis que UI représente les unités internationales pour l'interleukine-2 indiquées dans la 1<sup>ére</sup> norme internationale pour la IL-2 humaine, 86/504 de l'organisation mondiale de la santé.

13. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 3200 UI, tandis que UI représente les unités internationales pour l'interleukine-2 indiquées dans la 1<sup>ère</sup> norme internationale pour la IL-2 humaine, 86/504 de l'organisation mondiale de la santé.

14. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 4800 UI, tandis que UI représente les unités internationales pour l'interleukine-2 indiquées dans la 1<sup>ère</sup> norme internationale pour la IL-2 humaine, 86/504 de l'organisation mondiale de la santé.

15. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 8000 UI, tandis que UI représente les unités internationales pour l'interleukine-2 indiquées dans la 1<sup>ère</sup> norme internationale pour la IL-2 humaine, 86/504 de l'organisation mondiale de la santé.

16. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 9600 UI, tandis que UI représente les unités internationales pour l'interleukine-2 indiquées dans la 1<sup>ère</sup> norme internationale pour la IL-2 humaine, 86/504 de l'organisation mondiale de la santé.

17. Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de deux semaines à raison de 12000 UI, tandis que UI représente les unités internationales pour l'interleukine-2 indiquées dans la 1<sup>ère</sup> norme internationale pour la IL-2 humaine, 86/504 de l'organisation mondiale de la santé.

18. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 200 UI, tandis que UI représente les unités internationales pour l'interleukine-2 indiquées dans la 1<sup>ére</sup> norme internationale pour la IL-2 humaine, 86/504 de l'organisation mondiale de la santé.

19. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 400 UI, tandis que UI représente les unités internationales pour l'interleukine-2 indiquées dans la 1<sup>ére</sup> norme internationale pour la IL-2 humaine, 86/504 de l'organisation mondiale de la santé.

20. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 800 UI, tandis que UI représente les unités internationales pour l'interleukine-2 indiquées dans la 1<sup>ère</sup> norme internationale pour la IL-2 humaine, 86/504 de l'organisation mondiale de la santé.

21. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 1200 UI, tandis que UI représente les unités internationales pour l'interleukine-2 indiquées dans la 1<sup>ère</sup> norme internationale pour la IL-2 humaine, 86/504 de l'organisation mondiale de la santé.

22. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 1600 UI, tandis que UI représente les unités internationales pour l'interleukine-2 indiquées dans la 1<sup>ère</sup> norme internationale pour la IL-2 humaine, 86/504 de l'organisation mondiale de la santé.

23. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 2400 UI, tandis que UI représente les unités internationales pour l'interleukine-2 indiquées dans la 1<sup>ère</sup> norme internationale pour la IL-2 humaine, 86/504 de l'organisation mondiale de la santé.

24. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 3200 UI, tandis que UI représente les unités internationales pour l'interleukine-2 indiquées dans la 1<sup>ère</sup> norme internationale pour la IL-2 humaine, 86/504 de l'organisation mondiale de la santé.

25. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 4800 UI, tandis que UI représente les unités internationales pour l'interleukine-2 indiquées dans la 1<sup>ère</sup> norme internationale pour la IL-2 humaine, 86/504 de l'organisation mondiale de la santé.

26. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 8000 UI, tandis que UI représente les unités internationales pour l'interleukine-2 indiquées dans la 1<sup>ére</sup> norme internationale pour la IL-2 humaine, 86/504 de l'organisation mondiale de la santé.

27. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 9600 UI, tandis que UI représente les unités internationales pour l'interleukine-2 indiquées dans la 1<sup>ère</sup> norme internationale pour la IL-2 humaine, 86/504 de l'organisation mondiale de la santé.

28. Mélange de cytokines exempt de sérum et exempt d'agents mitogènes selon la revendication 1, dans lequel ledit mélange de cytokines exempt de sérum et exempt d'agents mitogènes est administré cinq fois par semaine sur une période de trois semaines à raison de 12000 UI, tandis que UI représente les unités internationales pour l'interleukine-2 indiquées dans la 1<sup>ère</sup> norme internationale pour la IL-2 humaine, 86/504 de l'organisation mondiale de la santé.

**29.** Mélange de cytokines exempt de sérum et exempt d'agents mitogènes selon la revendication 1, dans lequel ledit mélange de cytokines exempt de sérum et exempt d'agents mitogènes est administré trois fois par semaine sur une période de huit à vingt-six semaines dans un intervalle d'environ 20 UI à 1600 UI, tandis que UI représente les unités internationales pour l'interleukine-2 indiquées dans la 1ère norme internationale pour la IL-2 humaine, 86/504 de l'organisation mondiale de la santé.

**30.** Mélange de cytokines exempt de sérum et exempt d'agents mitogènes selon la revendication 3, dans lequel ledit mélange de cytokines exempt de sérum et exempt d'agents mitogènes est administré cinq fois par semaine sur une période de huit à vingt-six semaines dans un intervalle d'environ 20 UI à 1600 UI, tandis que UI représente les unités internationales pour l'interleukine-2 indiquées dans la 1ère norme internationale pour la IL-2 humaine, 86/504 de l'organisation mondiale de la santé.

**31.** Mélange de cytokines exempt de sérum et exempt d'agents mitogènes selon la revendication 1, dans lequel la maladie ou le trouble est choisi dans le groupe consistant en affection cardiovasculaire, dyslipidémie, dyslipidoprotéinémie, hypertension, et hyperlipidémie.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0374791 A
- US 5945097 A
- WO 02083076 A
- US 5093479 A
- US 4390623 A
- US 4388309 A
- US 4406830 A
- US 4661447 A
- US 4681844 A
- US 4464355 A

**Non-patent literature cited in the description**

- **Reaven.** *Annu. Rev. Med.,* 1993, vol. 44, 121-131
- **Badimon et al.** *Circulation,* 1992, vol. 86 (111), 86-94
- **Dansky ; Fisher.** *Circulation,* 1999, vol. 100, 1762-3
- **Brown ; Goldstein.** The Pharmacological Basis Of Therapeutics. Pergamon Press, 1990, 874-896
- National Cholesterol Education Program Expert Panel. Second Report of the Expert Panel on Detection, Evaluation, and Treatment of High Blood Cholesterol in Adults. *Circulation,* 1994, vol. 89, 1329-445
- Handbook of Statistical Methods for Engineers and Scientists. McGraw Hill, 1990
- Statistical Operations Analysis of Health Research Data. Blackwell Science Inc, 1996
- **Hadden et al.** Mixed Interleukins and Thymosin Fraction V Synergistically Induce T Lymphocyte Development in Hydrocortisone-Treated Aged Mice. *Cell. Immunol.,* 1992, vol. 144, 228-236
- **Golumbek et al.** Treatment of Established Renal Cancer by Tumor Cells Engineered to Secrete Interleukin-4. *Science,* 1991, vol. 254, 713-716